(19) Europäisches Patentamt
European Patent Office
Office européen des brevets

(11) Publication number: **0 353 464 B1**

(12) **EUROPEAN PATENT SPECIFICATION**

(45) Date of publication of patent specification: **20.10.93**

(51) Int. Cl.5: **C12N 15/53**, C07H 21/04, C12N 9/02

(21) Application number: **89111958.8**

(22) Date of filing: **30.06.89**

(54) **Luciferase gene and novel recombinant DNA as well as a method for production of luciferase.**

(30) Priority: **01.07.88 JP 162402/88**
**22.12.88 JP 322029/88**

(43) Date of publication of application:
**07.02.90 Bulletin 90/06**

(45) Publication of the grant of the patent:
**20.10.93 Bulletin 93/42**

(84) Designated Contracting States:
**CH DE FR GB LI NL**

(56) References cited:
**EP-A- 0 273 889**
**EP-A- 0 301 541**
**WO-A-87/03304**
**US-A- 4 581 335**

(73) Proprietor: **KIKKOMAN CORPORATION**
**339 Noda**
**Noda-shi(JP)**

(72) Inventor: **Tatsumi, Hiroki**
**Kikkoman Apartment B-306**
**65-1, Yanagisawa Noda-shi(JP)**
Inventor: **Kajiyama, Naoki**
**101-2, Miyazaki**
**Noda-shi(JP)**
Inventor: **Nakano, Eiichi**
**2-86, Kizora**
**Iwatsuki-shi(JP)**

(74) Representative: **Kolb, Helga, Dr. Dipl.-Chem. et al**
**Hoffmann, Eitle & Partner,**
**Patentanwälte,**
**Postfach 81 04 20**
**D-81904 München (DE)**

EP 0 353 464 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

1. Field of the Invention

This invention relates to a luciferase gene derived from Luciola lateralis (HEIKE firefly) and a novel recombinant DNA having integrated the gene therein as well as a method of producing a luciferase using the recombinant DNA.

2. Description of the Prior Art

Luciferase from fireflies belonging to the genus Luciola is merely obtained by isolating and purifying from the collected fireflies belonging to the genus Luciola [Proc. Natl. Acad. Sci., 74 (7), 2799-2802 (1977)].

Luciferases are very effectively usable, e.g., for quantitative determination of ATP.

Since luciferases described above are derived from insects, however, fireflies belonging to the genus Luciola must be collected from the natural world to produce luciferases; alternatively, such fireflies must be cultivated and luciferases should be isolated and refined from the fireflies so that much time and labors are required for the production.

As a result of various investigations to solve the foregoing problems, the present inventors have found that by producing a recombinant DNA by inserting DNA containing a Luciola lateralis-derived luciferase-coding gene into a vector DNA and culturing in a medium a luciferase-producing microorganism belonging to the genus Escherichia and bearing the recombinant DNA, luciferase can be efficiently produced in a short period of time. As a result of further investigations on luciferase gene derived from Luciola lateralis, the present inventors have also succeeded in isolating a luciferase gene derived from Luciola lateralis and determining its structure, for the first time. This invention has thus been accomplished.

SUMMARY OF THE INVENTION

According to this invention, there are provided:

(1) A Luciola lateralis-derived luciferase gene defined by a restriction enzyme map described below:

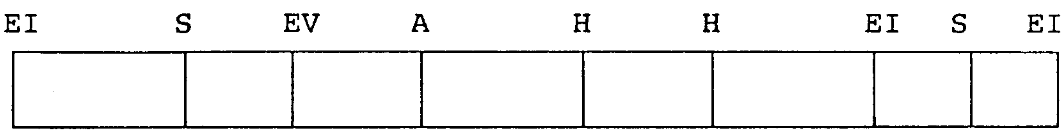


wherein EI represents Eco RI, S represents Ssp I, EV represents Eco RV, A represents Apa I and H represents Hpa I.

(2) A luciferase gene according to (1) which encodes an amino acid sequence shown below:

| | | | | | | | | | |
|---|---|---|---|---|---|---|---|---|---|
| | | | | | | | | | 10 |
| Met | Glu | Asn | Met | Glu | Asn | Asp | Glu | Asn | Ile |
| | | | | | | | | | 20 |
| Val | Tyr | Gly | Pro | Glu | Pro | Phe | Tyr | Pro | Ile |
| | | | | | | | | | 30 |
| Glu | Glu | Gly | Ser | Ala | Gly | Ala | Gln | Leu | Arg |
| | | | | | | | | | 40 |
| Lys | Tyr | Met | Asp | Arg | Tyr | Ala | Lys | Leu | Gly |
| | | | | | | | | | 50 |
| Ala | Ile | Ala | Phe | Thr | Asn | Ala | Leu | Thr | Gly |
| | | | | | | | | | 60 |
| Val | Asp | Tyr | Thr | Tyr | Ala | Glu | Tyr | Leu | Glu |
| | | | | | | | | | 70 |
| Lys | Ser | Cys | Cys | Leu | Gly | Glu | Ala | Leu | Lys |
| | | | | | | | | | 80 |
| Asn | Tyr | Gly | Leu | Val | Val | Asp | Gly | Arg | Ile |
| | | | | | | | | | 90 |
| Ala | Leu | Cys | Ser | Glu | Asn | Cys | Glu | Glu | Phe |
| | | | | | | | | | 100 |
| Phe | Ile | Pro | Val | Leu | Ala | Gly | Leu | Phe | Ile |
| | | | | | | | | | 110 |
| Gly | Val | Gly | Val | Ala | Pro | Thr | Asn | Glu | Ile |
| | | | | | | | | | 120 |
| Tyr | Thr | Leu | Arg | Glu | Leu | Val | His | Ser | Leu |
| | | | | | | | | | 130 |
| Gly | Ile | Ser | Lys | Pro | Thr | Ile | Val | Phe | Ser |
| | | | | | | | | | 140 |
| Ser | Lys | Lys | Gly | Leu | Asp | Lys | Val | Ile | Thr |
| | | | | | | | | | 150 |
| Val | Gln | Lys | Thr | Val | Thr | Ala | Ile | Lys | Thr |
| | | | | | | | | | 160 |
| Ile | Val | Ile | Leu | Asp | Ser | Lys | Val | Asp | Tyr |
| | | | | | | | | | 170 |
| Arg | Gly | Tyr | Gln | Ser | Met | Asp | Asn | Phe | Ile |
| | | | | | | | | | 180 |
| Lys | Lys | Asn | Thr | Pro | Gln | Gly | Phe | Lys | Gly |
| | | | | | | | | | 190 |
| Ser | Ser | Phe | Lys | Thr | Val | Glu | Val | Asn | Arg |

200
Lys Glu Gln Val Ala Leu Ile Met Asn Ser
210
Ser Gly Ser Thr Gly Leu Pro Lys Gly Val
220
Gln Leu Thr His Glu Asn Ala Val Thr Arg
230
Phe Ser His Ala Arg Asp Pro Ile Tyr Gly
240
Asn Gln Val Ser Pro Gly Thr Ala Ile Leu
250
Thr Val Val Pro Phe His His Gly Phe Gly
260
Met Phe Thr Thr Leu Gly Tyr Leu Thr Cys
270
Gly Phe Arg Ile Val Met Leu Thr Lys Phe
280
Asp Glu Glu Thr Phe Leu Lys Thr Leu Gln
290
Asp Tyr Lys Cys Ser Ser Val Ile Leu Val
300
Pro Thr Leu Phe Ala Ile Leu Asn Arg Ser
310
Glu Leu Leu Asp Lys Tyr Asp Leu Ser Asn
320
Leu Val Glu Ile Ala Ser Gly Gly Ala Pro
330
Leu Ser Lys Glu Ile Gly Glu Ala Val Ala
340
Arg Arg Phe Asn Leu Pro Gly Val Arg Gln
350
Gly Tyr Gly Leu Thr Glu Thr Thr Ser Ala
360
Ile Ile Ile Thr Pro Glu Gly Asp Asp Lys
370
Pro Gly Ala Ser Gly Lys Val Val Pro Leu
380
Phe Lys Ala Lys Val Ile Asp Leu Asp Thr

| | | | | | | | | | |
|---|---|---|---|---|---|---|---|---|---|
| | | | | | | | | | 390 |
| Lys | Lys | Thr | Leu | Gly | Pro | Asn | Arg | Arg | Gly |
| | | | | | | | | | 400 |
| Glu | Val | Cys | Val | Lys | Gly | Pro | Met | Leu | Met |
| | | | | | | | | | 410 |
| Lys | Gly | Tyr | Val | Asp | Asn | Pro | Glu | Ala | Thr |
| | | | | | | | | | 420 |
| Arg | Glu | Ile | Ile | Asp | Glu | Glu | Gly | Trp | Leu |
| | | | | | | | | | 430 |
| His | Thr | Gly | Asp | Ile | Gly | Tyr | Tyr | Asp | Glu |
| | | | | | | | | | 440 |
| Glu | Lys | His | Phe | Phe | Ile | Val | Asp | Arg | Leu |
| | | | | | | | | | 450 |
| Lys | Ser | Leu | Ile | Lys | Tyr | Lys | Gly | Tyr | Gln |
| | | | | | | | | | 460 |
| Val | Pro | Pro | Ala | Glu | Leu | Glu | Ser | Val | Leu |
| | | | | | | | | | 470 |
| Leu | Gln | His | Pro | Asn | Ile | Phe | Asp | Ala | Gly |
| | | | | | | | | | 480 |
| Val | Ala | Gly | Val | Pro | Asp | Pro | Ile | Ala | Gly |
| | | | | | | | | | 490 |
| Glu | Leu | Pro | Gly | Ala | Val | Val | Val | Leu | Glu |
| | | | | | | | | | 500 |
| Lys | Gly | Lys | Ser | Met | Thr | Glu | Lys | Glu | Val |
| | | | | | | | | | 510 |
| Met | Asp | Tyr | Val | Ala | Ser | Gln | Val | Ser | Asn |
| | | | | | | | | | 520 |
| Ala | Lys | Arg | Leu | Arg | Gly | Gly | Val | Arg | Phe |
| | | | | | | | | | 530 |
| Val | Asp | Glu | Val | Pro | Lys | Gly | Leu | Thr | Gly |
| | | | | | | | | | 540 |
| Lys | Ile | Asp | Gly | Lys | Ala | Ile | Arg | Glu | Ile |
| Leu | Lys | Lys | Pro | Val | Ala | Lys | Met | | |

(3) A luciferase gene according to (1) or (2) which is represented by a nucleotide sequence shown below.

```
                                                30
ATG GAA AAC ATG GAG AAC GAT GAA AAT ATT
                                                60
GTG TAT GGT CCT GAA CCA TTT TAC CCT ATT
                                                90
GAA GAG GGA TCT GCT GGA GCA CAA TTG CGC
                                               120
AAG TAT ATG GAT CGA TAT GCA AAA CTT GGA
                                               150
GCA ATT GCT TTT ACT AAC GCA CTT ACC GGT
                                               180
GTC GAT TAT ACG TAC GCC GAA TAC TTA GAA
                                               210
AAA TCA TGC TGT CTA GGA GAG GCT TTA AAG
                                               240
AAT TAT GGT TTG GTT GTT GAT GGA AGA ATT
                                               270
GCG TTA TGC AGT GAA AAC TGT GAA GAA TTC
                                               300
TTT ATT CCT GTA TTA GCC GGT TTA TTT ATA
                                               330
GGT GTC GGT GTG GCT CCA ACT AAT GAG ATT
                                               360
TAC ACT CTA CGT GAA TTG GTT CAC AGT TTA
                                               390
GGC ATC TCT AAG CCA ACA ATT GTA TTT AGT
                                               420
TCT AAA AAA GGA TTA GAT AAA GTT ATA ACT
                                               450
GTA CAA AAA ACG GTA ACT GCT ATT AAA ACC
                                               480
ATT GTT ATA TTG GAC AGC AAA GTG GAT TAT
                                               510
AGA GGT TAT CAA TCC ATG GAC AAC TTT ATT
                                               540
AAA AAA AAC ACT CCA CAA GGT TTC AAA GGA
                                               570
TCA AGT TTT AAA ACT GTA GAA GTT AAC CGC
                                               600
AAA GAA CAA GTT GCT CTT ATA ATG AAC TCT
                                               630
TCG GGT TCA ACC GGT TTG CCA AAA GGT GTG
```

```
                                                660
CAA CTT ACT CAT GAA AAT GCA GTC ACT AGA
                                                690
TTT TCT CAC GCT AGA GAT CCA ATT TAT GGA
                                                720
AAC CAA GTT TCA CCA GGC ACG GCT ATT TTA
                                                750
ACT GTA GTA CCA TTC CAT CAT GGT TTT GGT
                                                780
ATG TTT ACT ACT TTA GGC TAT CTA ACT TGT
                                                810
GGT TTT CGT ATT GTC ATG TTA ACG AAA TTT
                                                840
GAC GAA GAG ACT TTT TTA AAA ACA CTG CAA
                                                870
GAT TAC AAA TGT TCA AGC GTT ATT CTT GTA
                                                900
CCG ACT TTG TTT GCA ATT CTT AAT AGA AGT
                                                930
GAA TTA CTC GAT AAA TAT GAT TTA TCA AAT
                                                960
TTA GTT GAA ATT GCA TCT GGC GGA GCA CCT
                                                990
TTA TCT AAA GAA ATT GGT GAA GCT GTT GCT
                                               1020
AGA CGT TTT AAT TTA CCG GGT GTT CGT CAA
                                               1050
GGC TAT GGT TTA ACA GAA ACA ACC TCT GCA
                                               1080
ATT ATT ATC ACA CCG GAA GGC GAT GAT AAA
                                               1110
CCA GGT GCT TCT GGC AAA GTT GTG CCA TTA
                                               1140
TTT AAA GCA AAA GTT ATC GAT CTT GAT ACT
                                               1170
AAA AAA ACT TTG GGC CCG AAC AGA CGT GGA
                                               1200
GAA GTT TGT GTA AAG GGT CCT ATG CTT ATG
                                               1230
AAA GGT TAT GTA GAT AAT CCA GAA GCA ACA
                                               1260
AGA GAA ATC ATA GAT GAA GAA GGT TGG TTG
                                               1290
CAC ACA GGA GAT ATT GGG TAT TAC GAT GAA
```

```
                                          1320
GAA AAA CAT TTC TTT ATC GTG GAT CGT TTG
                                          1350
AAG TCT TTA ATC AAA TAC AAA GGA TAT CAA
                                          1380
GTA CCA CCT GCT GAA TTA GAA TCT GTT CTT
                                          1410
TTG CAA CAT CCA AAT ATT TTT GAT GCC GGC
                                          1440
GTT GCT GGC GTT CCA GAT CCT ATA GCT GGT
                                          1470
GAG CTT CCG GGA GCT GTT GTT GTA CTT GAA
                                          1500
AAA GGA AAA TCT ATG ACT GAA AAA GAA GTA
                                          1530
ATG GAT TAC GTT GCT AGT CAA GTT TCA AAT
                                          1560
GCA AAA CGT TTG CGT GGT GGT GTC CGT TTT
                                          1590
GTG GAC GAA GTA CCT AAA GGT CTC ACT GGT
                                          1620
AAA ATT GAC GGT AAA GCA ATT AGA GAA ATA

CTG AAG AAA CCA GTT GCT AAG ATG
```

According to this invention, there is also provided a novel recombinant DNA obtained by inserting a gene coding for luciferase derived from Luciola lateralis into a vector DNA. According to this invention, there is further provided a method of producing a luciferase which comprises culturing in a medium a microorganism belonging to the genus Escherichia and bearing a recombinant DNA obtained by inserting a gene coding for luciferase derived from Luciola lateralis in a vector DNA, and collecting a luciferase from the culture.

## BRIEF DESCRIPTION OF THE DRAWINGS

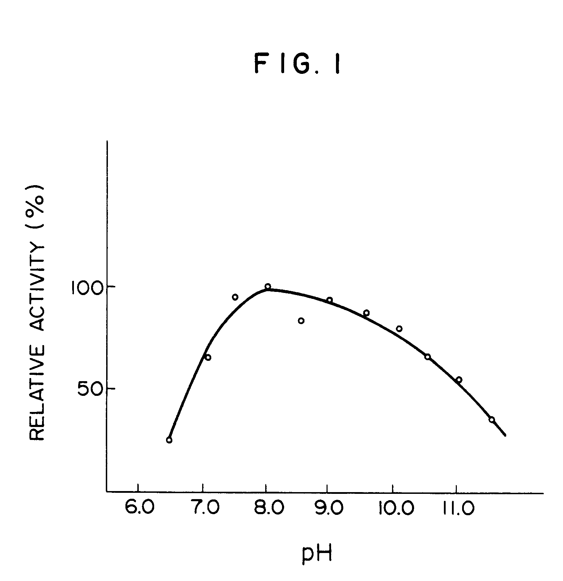

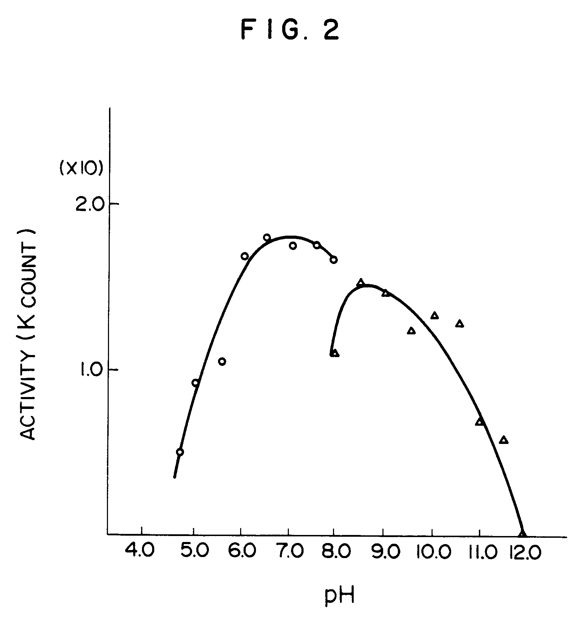

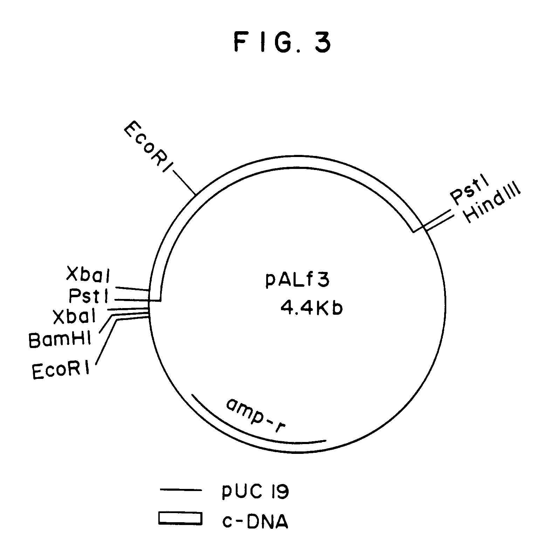

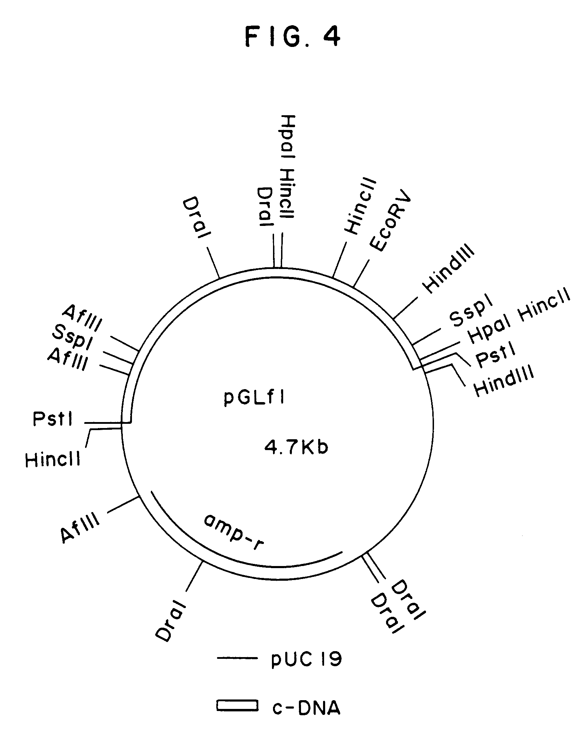

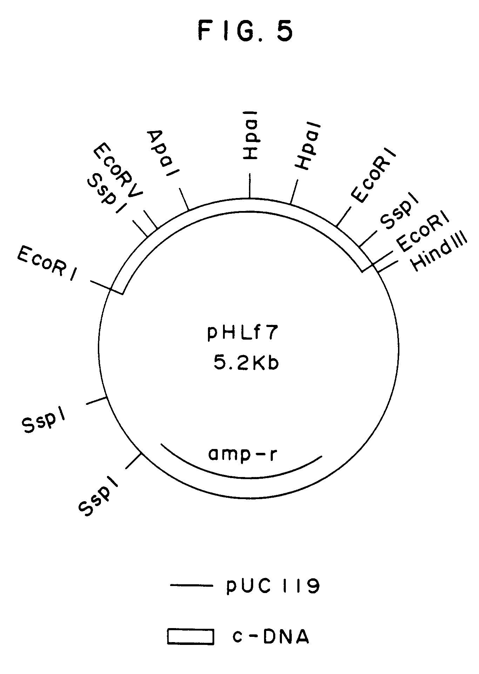

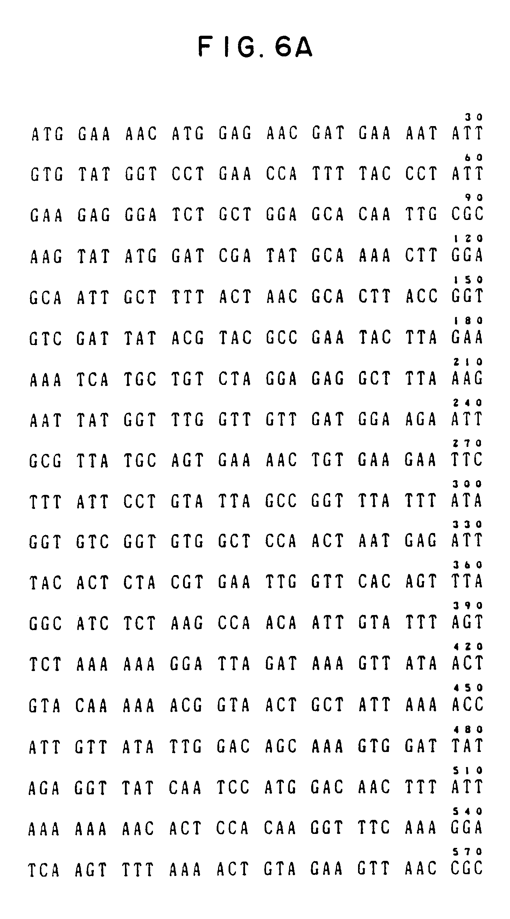

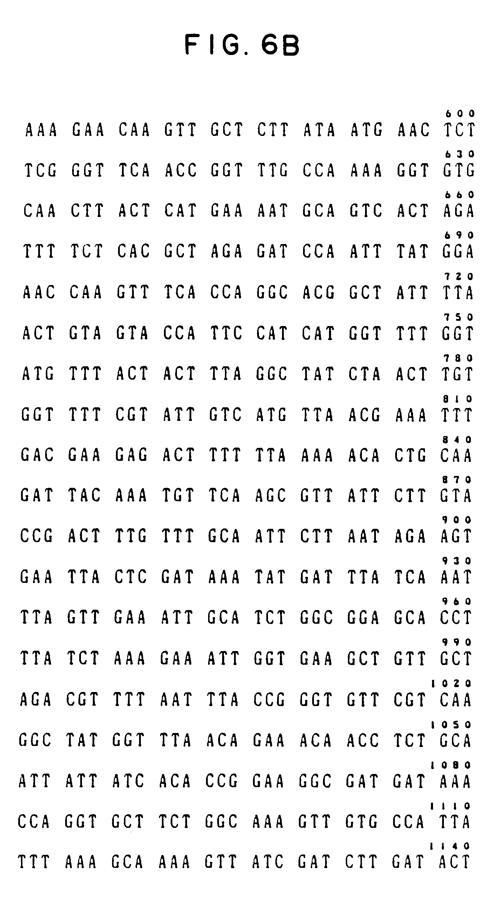

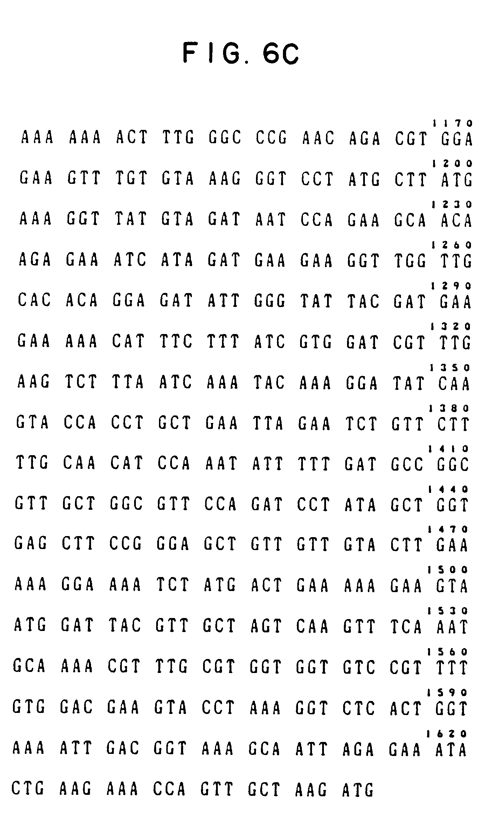

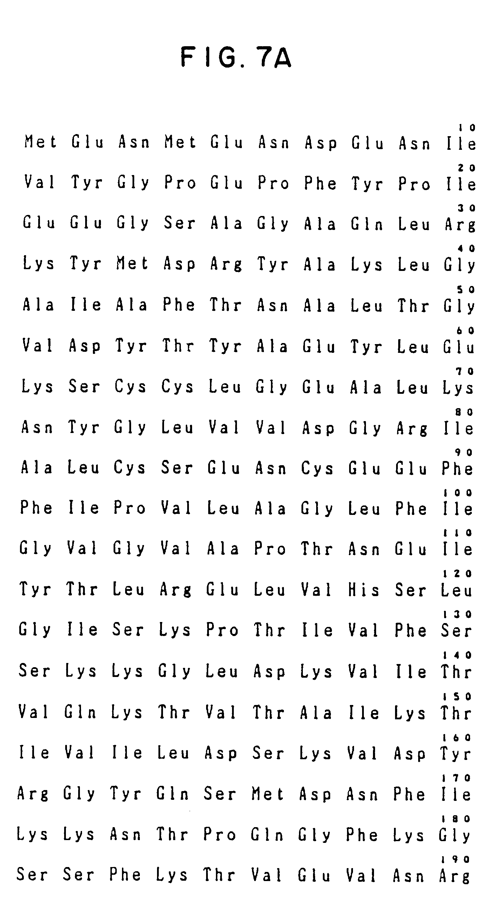

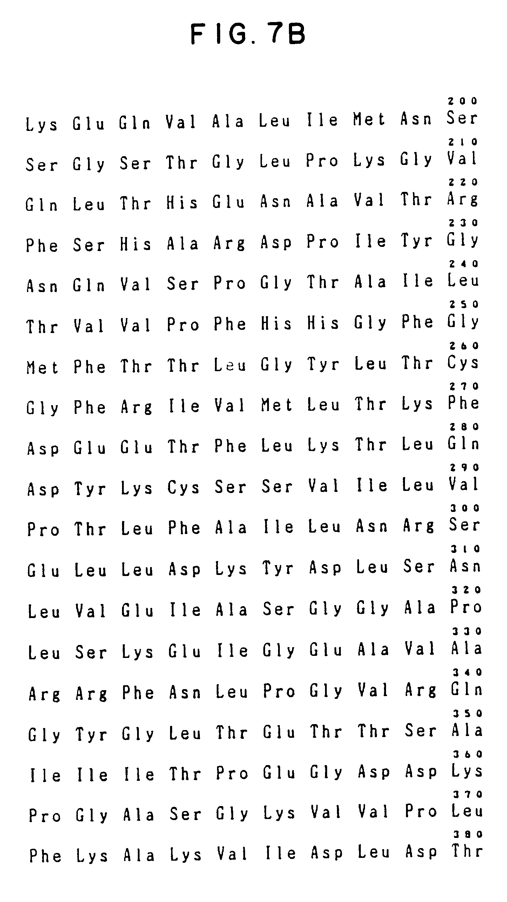

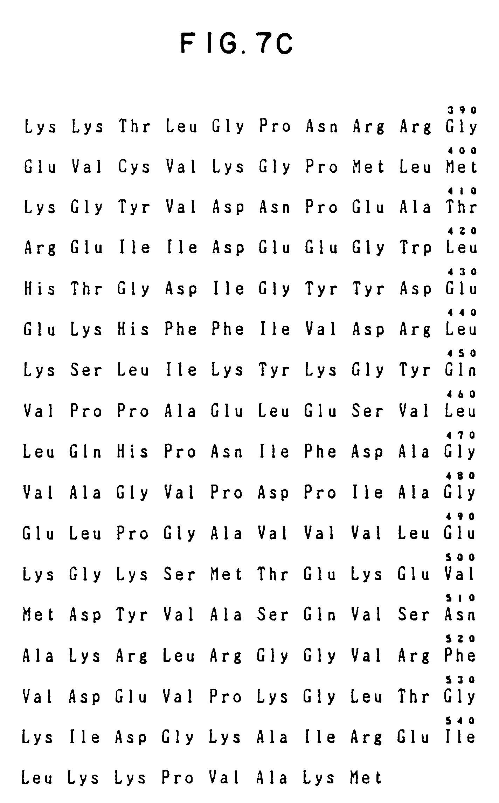

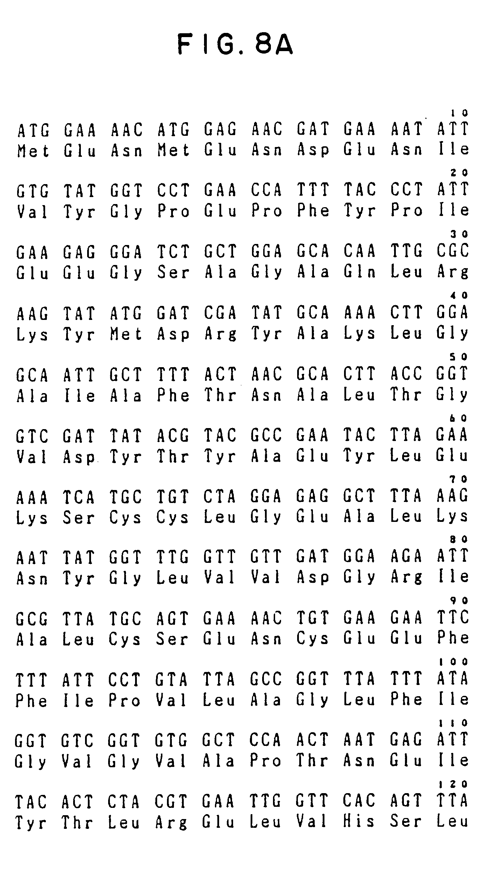

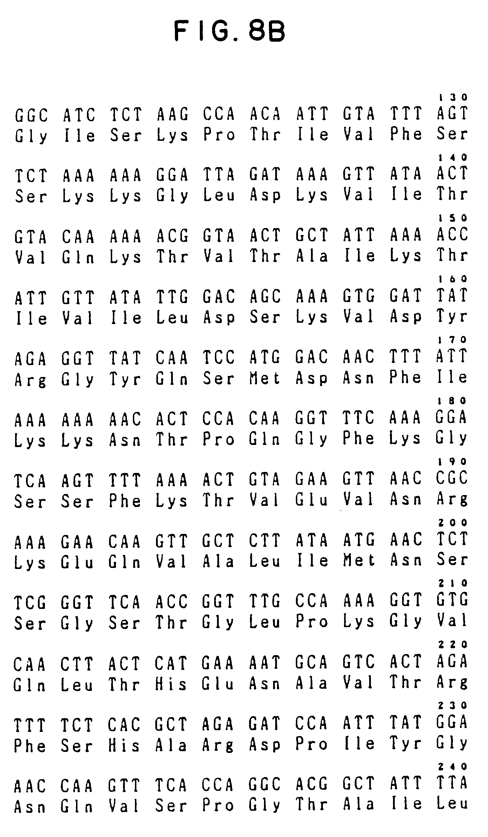

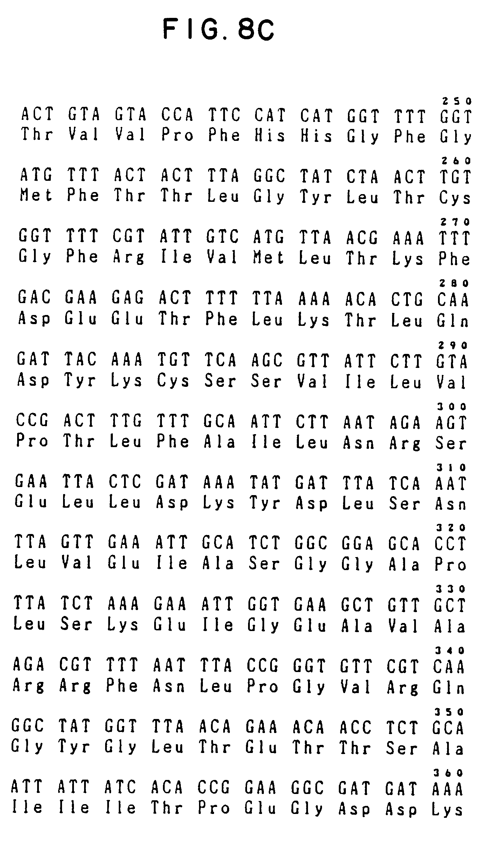

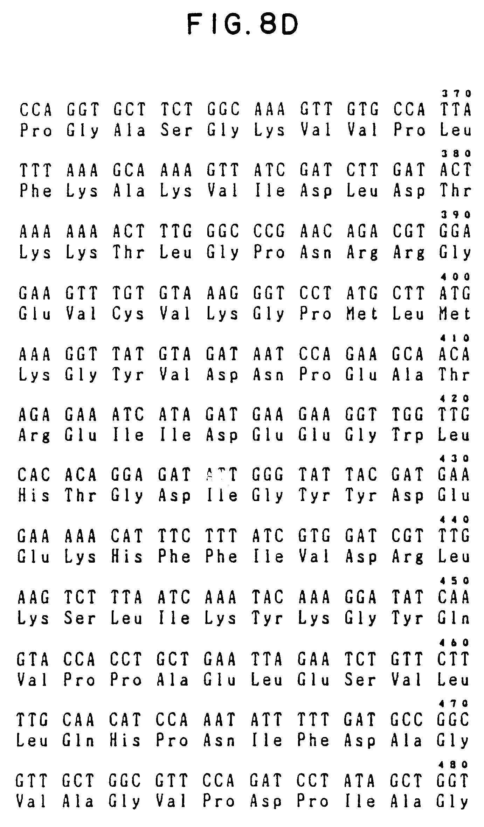

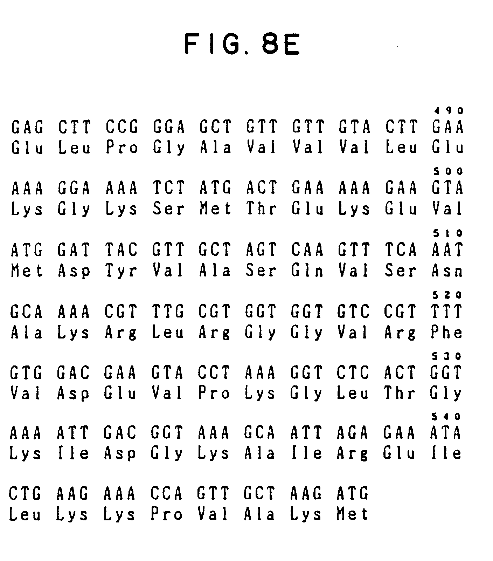

Fig. 1 shows an optimum pH range of luciferase derived from Luciola lateralis. Fig. 2 shows a stable pH range of luciferase derived from Luciola lateralis. Fig. 3 shows a cleavage map of recombinant plasmid pALf3 DNA with restriction enzymes. Fig. 4 shows a cleavage map of recombinant plasmid pGLf1 DNA with restriction enzymes. Fig. 5 shows a cleavage map of recombinant plasmid pHLf7 DNA with restriction enzymes. Fig. 6 shows a nucleotide sequence of Luciola lateralis-derived luciferase gene in accordance with this invention. Fig. 7 shows an amino acid sequence of polypeptide translated from Luciola lateralis-derived luciferase gene of this invention. Fig. 8 shows a nucleotide sequence of Luciola lateralis-derived luciferase gene and the amino acid sequence corresponding thereto.

## DETAILED DESCRIPTION OF THE PREFERRED EMBODIMENTS

Hereafter this invention is described in detail.

In survey of DNA bearing a gene encoding luciferase derived from Luciola lateralis (HEIKE firefly), DNA containing a gene encoding luciferase derived from Luciola cruciata (GENJI firefly) belonging to the same genus, which is one of fireflies, is used as a probe; further in survey of DNA bearing a Luciola cruciata-derived luciferase-coding gene, DNA containing a gene coding for luciferase derived from Photinus pyralis - (American firefly), which is one of fireflies, is used as a probe.

Therefore, production of the gene encoding luciferase from Photinus pyralis is firstly described below. Subsequently, production of the gene encoding luciferase from Luciola cruciata is described and finally, production of the gene encoding luciferase from Luciola lateralis is described.

To prepare m-RNA from the posterior portion of Photinus pyralis which is one of fireflies, m-RNA can be obtained according to methods described in, for example, Molecular Cloning, page 196, Cold Spring Harbor Laboratory (1982), Haruo Ozeki and Reiro Shimura, BUNSHI IDENGAKU JIKKENHO (Experimental Molecular Genetics), pages 66-67 (1983), etc.

Concentration of m-RNA coding for luciferase from the obtained m-RNA can be performed by a method described in, for example, Biomedical Research, 3, 534-540 (1982) or the like.

In this case, anti-luciferase serum to luciferase is used. This serum can be obtained by, for example, Yuichi Yamamura, MEN-EKI KAGAKU (Immunochemistry), pages 43-50 (1973), etc.

Synthesis of c-DNA from the m-RNA coding for luciferase can be performed by methods described in, for example, Mol. Cell Biol., 2, 161 (1982) and Gene, 25, 263 (1983).

Then, the thus obtained c-DNA is integrated into, for example, plasmid pMCE10 DNA {plasmid produced using plasmid pKN305 [plasmid having a promoter of Escherichia coli tryptophan operator described in Agr. Biol. Chem., 50, 271 (1986)] and plasmid pMC1843 [plasmid containing Escherichia coli $\beta$-galactosidase structural gene described in Methods in Enzymology, 100, 293-308 (1983)]}, etc. to produce various recombinant plasmid DNAs. Using these DNAs, transformation of Escherichia coli (E. coli) DH1 (ATCC 33849), E. coli HB101 (ATCC 33694), etc. is effected by the method of Hanahan [DNA Cloning, 1, 109-135 (1985)] to obtain various transformants.

The recombinant plasmid c-DNAs possessed by the thus obtained transformants are plasmids wherein c-DNA has been integrated in the middle of E. coli $\beta$-galactosidase structural gene. A peptide encoded by c-DNA is expressed as a protein fused with $\beta$-galactosidase.

In order to detect c-DNA coding for luciferase from the various transformants described above, the transformants are cultured thereby to express cell protein. By determining if any protein crossing over anti-luciferase serum is present, its detection can be made. Methods described in, for example, Agric. Biol. Chem., 50, 271 (1986) and Anal. Biochem., 112, 195 (1981), etc. can be used for the detection.

Next, after labeling c-DNA of incomplete luciferase with $^{32}P$ by the nick translation method [Molecular Cloning, pages 109-112, Cold spring Harbor Laboratory (1982) and J. Mol. Biol., 113, 237-251 (1977)], using the colony hybridization method [Protein, Nucleic Acid & Enzyme, 26, 575-579 (1981)], an Escherichia coli strain having plasmid DNA containing Photinus pyralis luciferase c-DNA of 1.8 Kb can be obtained from a Photinus pyralis-derived c-DNA library prepared using plasmid pUC19 DNA (manufactured by Takara Shuzo Co., Ltd.) as a vector.

To obtain a DNA containing the gene coding for luciferase derived from Photinus pyralis from the thus obtained recombinant plasmid DNA, restriction enzymes, e.g., Eco RI and Cla I, are acted on the plasmid DNA at temperatures of 30 to 40 °C, preferably at 37 °C, for 1 to 24 hours, preferably for 2 hours; the solution obtained after completion of the reaction is subjected to agarose gel electrophoresis [which is described in Molecular Cloning, page 150, Cold Spring Harbor Laboratory (1982)] to obtain the DNA containing the gene coding for luciferase derived from Photinus pyralis.

Next, production of the Luciola cruciata-derived luciferase-coding gene are described below.

Preparation of m-RNA from the posterior portion of Luciola cruciata and synthesis of c-DNA from the m-RNA can be conducted, for example, in quite the same manner as in the preparation of the m-RNA of Photinus pyralis and synthesis of the c-DNA described above.

Then, the thus obtained c-DNA is integrated into a vector DNA, for example, plasmid pUC19 DNA (manufactured by Takara Shuzo Co., Ltd.), etc. to obtain various recombinant plasmid DNAs. Using these DNAs, transformation of E. coli DH1 (ATCC 33849), E. coli HB101 (ATCC 33694), etc. is effected by the method of Hanahan [DNA Cloning, 1, 109-135 (1985)] to obtain various transformants.

Next, after labeling c-DNA of luciferase derived from Photinus pyralis with $^{32}P$ by the nick translation method [Molecular Cloning, pages 109-112, Cold Spring Harbor Laboratory (1982) and J. Mol. Biol., 113, 237-251 (1971)], using the colony hybridization method [Protein, Nucleic Acid & Enzyme, 26, 575-579 (1981)], an Escherichia coli strain having plasmid DNA containing Luciola cruciata luciferase c-DNA of 2.0 Kb can be obtained from a Luciola cruciata-derived c-DNA library prepared using plasmid pUC19 DNA (manufactured by Takara Shuzo Co., Ltd.) as a vector.

To obtain the purified plasmid DNA, there is used, for example, a method described in Proc. Natl. Acad. Sci., 62 1159-1166 (1969), etc.

By acting on the purified plasmid DNA, for example, restriction enzyme, e.g., Pst I (manufactured by Takara Shuzo Co., Ltd.) at a temperature of 30 °C to 40 °C, preferably at 37 °C for 1 to 24 hours, preferably for 2 hours, the resulting solution obtained after completion of the reaction is subjected to agarose gel

electrophoresis [which is described in Molecular Cloning, page 150, Cold Spring Harbor Laboratory (1982)] to obtain the DNA containing the gene coding for luciferase derived from Luciola cruciata.

Next, production of the Luciola lateralis-derived luciferase-coding gene in accordance with this invention are described below.

Firstly, as source from which the m-RNA coding for luciferase derived from Luciola lateralis is collected, the posterior portion of Luciola lateralis is preferred since the m-RNA is present in the posterior portion of this firefly.

Preparation of m-RNA from the posterior portion of the firefly and synthesis of c-DNA from the m-RNA can be conducted, for example, in quite the same manner as in the preparation of the m-RNA of Photinus pyralis and synthesis of the c-DNA described above.

Then, the thus obtained c-DNA is integrated into a vector DNA, for example, plasmid pUC119 DNA, etc. to obtain various recombinant plasmid DNAs. Using these DNAs, transformation of E. coli DH1 (ATCC 33849), E. coli HB101 (ATCC 33694), etc. is effected by the method of Hanahan [DNA Cloning, 1, 109-135 (1985)] to obtain various transformants.

Next, after labeling c-DNA of luciferase derived from Luciola cruciata with $^{32}P$ by the nick translation method [Molecular Cloning, pages 109-112, Cold Spring Harbor Laboratory (1982) and J. Mol. Biol., 113, 237-251 (1977)], using the colony hybridization method [Protein, Nucleic Acid & Enzyme, 26, 575-579 (1981)], an Escherichia coli stran having plasmid DNA containing Luciola lateralis luciferase c-DNA of 2.0 Kb can be obtained from a Luciola lateralis-derived c-DNA library prepared using plasmid pUC119 DNA (manufactured by Takara Shuzo Co., Ltd.) as a vector.

To obtain the purified recombinant DNA from the thus obtained microorganism, there is used, for example, a method described in Proc. Natl. Acad. Sci., 62 1159-1166 (1969), etc.

By acting on the purified, new recombinant DNA, for example, 2 units of restriction enzyme Eco RI (manufactured by Takara Shuzo Co., Ltd.) at a temperature of 30 °C or higher, preferably at 37 °C, for 1 to 4 hours, preferably for 2 hours, partial digestion is effected. Then, digestion product is subjected to agarose gel electrophoresis to obtain 2,000 bp DNA fragment containing all the gene coding for luciferase derived from Luciola lateralis.

On the other hand, a nucleotide sequence of this luciferase gene is determined by the method as shown in Item 18 in the example. The determined nucleotide sequence is shown in Fig. 6. Subsequently, an amino acid sequence of polypeptide translated from the nucleotide sequence is identified. The results are shown in Fig. 7.

The gene encoding the thus identified amino acid sequence is also included in this invention.

The above-mentioned microorgansim is then cultured in a medium and luciferase is collected from the culture.

Any medium may be used as far as it is used to culture microorganisms belonging to the genus Escherichia. Mention may be made of, for example, 1% (W/V) of trypton, 0.5% (W/V) of yeast extract, 0.5% (W/V) of NaCl and 1 mM of isopropyl-$\beta$-D-thiogalactoside, etc.

Temperature for the cultivation is between 30 and 40 °C, preferably about 37 °C and a time period for the cultivation is, for example, 4 to 8 hours, preferably about 4 hours.

The cells are collected from the culture by centrifugation at 8,000 r.p.m. for about 10 minutes. The obtained cells are homogenized by the method described in, for example, Methods in Enzymology, 133, 3-14 (1986) to obtain a crude enzyme solution.

The crude enzyme solution may be usable as it is; if necessary and desired, the crude enzyme solution can be purified by fractionation with ammonium sulfate, hydrophobic chromatography (for example, using BUTYL TOYOPEARL 650C, etc.), gel filtration (using, e.g., Ultrogel AcA34, etc.) thereby to give purified luciferase.

Physicochemical properties of the thus obtained luciferase are as described below.

(1) Action

The enzyme catalyzes the oxidation of luciferin by an oxygen molecule, as shown by the enzymatic reaction equation:

Present enzyme

Luciferin + ATP + $O_2$ ——→ Oxyluciferin + AMP + Pyrophosphoric acid + $O_2$ + light

(2) Substrate specificity

The enzyme does not act on ADP, CTP, UTP and GTP.

(3) Optimum pH, and pH range for stability

The optimum pH is, as shown in Fig. 1, 7.5 to 9.5 as measured by carrying out the reaction by the use of luciferin as a substrate at various pH values of 25 mM glycylglycine buffer solution in the range of 6.5 to 11.5 and at a temperature of 30 °C, and measuring the quantity of light (the number of photons) emitted in 20 seconds. The stable pH range of the enzyme is, as shown in Fig. 2, 6.0 to 10.5 as measured by adding the enzyme to each of buffer solutions [25 mM phosphate buffer solution (pH 4.6-8.0) and 25 mM glycine.sodium chloride-sodium hydroxide buffer solutions (pH 8.0 - 11.5), each of which contains ammonium sulfate to 10% saturation] containing luciferin, and allowing the enzyme to act at a temperature of 0 °C for 4 hours. In Fig. 2, ○—○ and △—△ show the activity in the case of using the 25 mM phosphate buffer solutions and the activity in the case of using 25 mM glycine.sodium chloride-sodium hydroxide buffer solutions, respectively.

(4) Measurement of titer

A luciferin mixed solution is prepared by mixing 8 ml of 25 mM glycylglycine buffer solution (pH 7.8), 0.5 ml of a magnesium sulfate solution [a solution prepared by adding magnesium sulfate to 25 mM glycylglycine buffer solution (pH 7.8), a magnesium sulfate concentration of 0.1 M] and 0.8 ml of a luciferin solution [a solution prepared adding luciferin to 25 mM glycylglycine buffer solution (pH 7.8), a luciferin concentration of 1 mM].

Into a mixture of 400 μl of the luciferin mixed solution thus obtained and 10 μl of luciferase to be assayed is poured 80 μl of an ATP solution [a solution prepared by adding ATP to 25 mM glycylglycine buffer solution (pH 7.8), an ATP concentration of 10 mM]. Simultaneously with the pouring, the number of photons generated is measured by adding up for 20 seconds by means of a luminometer (LUMINESCENCE READER BLR-201, manufactured by ALOKA Co., Ltd.).

(5) Ragne of temperature suitable for action

When the reaction is carried out at pH 7.8 at each temperature and the quantity of light (the number of photons) emitted in 20 seconds is measured, the suitable temperature for the action ranges from 0° to 50 °C.

(6) Conditions for inactivation by pH

At pH values of 5.0 or lower and 12.0 or higher, the enzyme is completely inactivated 4 hours after.

As is clear from the foregoing description, according to this invention, luciferase can be efficiently produced in an extremely short period of time, by culturing the microorgnism belonging to the genus Escherichia which contains the recombinant DNA having integrated therein the Luciola lateralis-derived luciferase gene of this invention. Therefore, this invention is extremely useful from an industrial point of view.

Hereafter this invention will be described in more detail by referring to the examples below.

Example

In Items 1 to 10 below, production of DNA containing a gene coding for luciferase of Photinus pyralis as one of fireflies (this DNA is used as a probe upon survey of DNA containing a gene coding for luciferase of Luciola cruciata) is described. Further in Items 11 to 13 below, production of DNA containing a gene coding for luciferase derived from Luciola cruciata (this DNA is used as a probe upon survey of DNA containing a gene coding for luciferase of Luciola lateralis) is described.

1. Preparation of m-RNA

Using a mortar and a pestle, 1 g of the dry posterior portion (manufactured by Sigma Co., Ltd.) of Photinus pyralis as one of fireflies was thoroughly ground, to which 5 ml of dissolution buffer [20 mM Tris-hydrochloride buffer (pH 7.4)/10 mM NaCl/3 mM magnesium acetate/5% (W/V) sucrose/1.2 % (V/V) Triton X-100/10 mM vanadyl nucleoside complex (manufactured by New England Biolab Co., Ltd.)] was added. The mixture was further ground as in the manner described above to give a solution containing the ground posterior portion of Photinus pyralis.

In a cup blender (manufactured by Nippon Seiki Seisakusho) was charged 5 ml of the thus obtained solution. After treating at 5,000 r.p.m. for 5 minutes, 12 ml of guanidine isothiocyanate solution (6M guanidine isothiocyanate/37.5 mM sodium citrate (pH 7.0)/0.75% (W/V) sodium N-lauroylsarcocine/0.15 M $\beta$-mercaptoethanol) was added to the system. The mixture was treated with the blender described above at 3,000 r.p.m. for 10 minutes. The resulting solution was filtered using a threefold gauze to give the filtrate. The filtrate was gently poured in layers onto 4 tubes for ultracentrifuging machine (manufactured by Hitachi Koki Co., Ltd.) in which 1.2 ml each of 5.7 M cesium chloride solution had previously be laid in layers. Using the ultracentrifuging machine (manufactured by Hitachi Koki Co., Ltd., SCP55H), centrifugation was performed at 30,000 r.p.m. for 16 hours to give precipitates.

The obtained precipitates were washed with cold 70% (V/V) ethanol and suspended in 4 ml of 10 mM Tris buffer [10 mM Tris-hydrochloride (pH 7.4)/5 mM EDTA/1% sodium dodecylsulfate]. The equal amount of a mixture of n-butanol and chloroform in 1 : 4 (volume ratio) was added to the mixture to perform extraction. The extract was centrifuged at 3,000 r.p.m. for 10 minutes in a conventional manner to separate into the aqueous phase and the organic solvent phase. To the organic solvent phase was added 4 ml of 10 mM Tris buffer described above. The procedure for the extraction and separation described above was repeated twice. To the aqueous phase obtained were added a 1/10 amount of 3 M sodium acetate (pH 5.2) and a 2-fold amount of cold ethanol were added. After allowing to stand at a temperature of -20 °C for 2 hours, the mixture was centrifuged at 8,000 r.p.m. for 20 minutes in a conventional manner to precipitate RNA. The obtained RNA was dissolved in 4 ml of water. After the operation for precipitation with ethanol described above was carried out, the obtained RNA was dissolved in 1 ml of water to give 3.75 mg of RNA.

By repeating the foregoing procedure again, 7 mg in total of RNA was prepared. To select m-RNA from the RNA, 7 mg of RNA was subjected to oligo(dT)-cellulose (manufactured by New England Biolab Co., Ltd.) column chromatography.

As the column, 2.5 ml of Terumo syringe (manufactured by Terumo Co., Ltd.) was used. After 0.5 g of resin was swollen with elution buffer [10 mM Tris-hydrochloride buffer (pH 7.6)/1 mM DETA/0.1% (W/V) sodium dodecylsulfate], the resin was packed in the column and equilibrated with binding buffer [10 mM Tris-hydrochloride (pH 7.6)/1 mM EDTA/0.4 M NaCl/0.1% (W/V) sodium dodecylsulfate].

To 7 mg of RNA was added the same amount of buffer [10 mM Tris-hydrochloride (pH 7.6)/1 mM EDTA/0.8 M NaCl/0.1% (W/V) sodium dodecylsulfate]. The mixture was heat-treated at a temperature of 65 °C for 10 minutes and then quenched in ice water. After subjecting to oligo(dT)-celluose column, the resin was washed with binding buffer to completely wash unbound r-RNA and t-RNA out. Further m-RNA was eluted with eluting buffer to give 40 $\mu$g of m-RNA.

2. Concentration of luciferase m-RNA

Next, the luciferase m-RNA was concentrated by sucrose density gradient centrifugation.

Sucrose density gradient of 10 to 25% (W/V) was produced by charging 0.5 ml of 40% (W/V) sucrose solution [50 mM Tris-hydrochloride buffer (pH 7.5)/20 mM NaCl/1 mM EDTA/40% (W/V) sucrose] in a polyaroma tube for Rotor SW41 manufactured by Beckmann Co., Ltd., laying 2.4 ml each of 25% (W/V), 20% (W/V), 15% (W/V) and 10% (W/V) of the sucrose solution in layers and allowing to stand the system at a temperature of 4 °C for 24 hours. To the sucrose density gradient, 30 $\mu$g of m-RNA was laid to form a layer. Using SW41 Rotor manufactured by Beckmann Co., Ltd., centrifugation was conducted at 30,000

r.p.m. at a temperature of 18 °C for 18 hours in a conventional manner. After the centrifuging operation, fractionation was performed by 0.5 ml each and m-RNA was recovered by the ethanol precipitation method. The m-RNA was dissolved in 10 µl of water.

Next, protein encoded by the m-RNA was examined, whereby the fraction concentrated on m-RNA of luciferase was identified. One microliter of the fractionated RNA, 9 µl of rabbit reticular erythrocyte lysate (manufactured by Amersham Co., Ltd.) and 1 µl of [$^{35}$S] methionine (manufactured by Amersham Co., Ltd.) were mixed and reacted at a temperature of 30 °C for 30 minutes. To the reaction mixture was added 150 µl of NET buffer [150 mM NaCl/5 mM EDTA/0.02% (W/V) $NaN_3$/20 mM Tris-hydrochloride buffer (pH 7.4)-/0.05% (W/V) Nonidet P-40 (manufactured by Besesda Research Laboratories Co., Ltd., surface active agent)] and, 1 µl of antiluciferase serum (produced as will be later described) was added to the mixture. After allowing to stand at a temperature of 20 °C for 30 hours, 10 mg of Protein A Sepharose (manufactured by Pharmacia Fine Chemicals Inc.) was added to the mixture. The resulting mixture was then centrifuged at 12,000 r.p.m. for a minute in a conventional manner to recover the resin.

The recovered resin was washed three times with 200 µl of NET buffer. To the resin was added 40 µl of sample buffer for SDS-PAGE [62.5 mM Tris-hydrochloride buffer (pH 6.8)/10% (V/V) glycerol/2% (W/V) sodium dodecylsulfate/5% (V/V) β-mercaptoethanol/0.02% (W/V) bromophenol blue]. The mixture was boiled at a temperature of 100 °C for 3 minutes and centrifuged at 12,000 r.p.m. for a minute in a conventional manner to recover the supernatant. The whole amount was applied onto 7.5% (W/V) sodium dodecylsulfate-polyacrylamide gel.

Gel electrophoresis was performed by the method of Laemmli [Nature, 227, 680 (1970)]. After the electrophoresis, the gel was immersed in 10% (V/V) acetic acid for 30 minutes to immobilize protein. Then, the gel was immersed in water for 30 minutes and further immersed in 1 M sodium salicylate solution for 30 minutes and then dried to give a dry gel. The dry gel was subjected to fluorography using an X ray film (manufactured by Fuji Photo Film Co., Ltd.; RX).

By the foregoing procedure, the band of luciferase protein was recognized on the X ray film only in the case of using the RNA from the fraction in which the luciferase m-RNA was present and, the fraction wherein the luciferase m-RNA was concentrated could be identified.

3. Production of anti-serum

Rabbit anti-luciferase serum to purified luciferase was produced by the following method.

A luciferase solution having a 3.2 mg/ml concentration [solution obtained by dissolving luciferase manufactured by Sigma Co., Ltd. in 0.5 M glycylglycine solution (pH 7.8)], 0.7 ml, was suspended in an equal amount of Freund's complete adjuvant. 2.24 mg of the suspension was administered as an antigen to the palm of Japanese white rabbit weighing 2 kg as an antigen. After feeding for 2 weeks, the same amount of antigen as in the initial amount was intracutaneously administered to the back. After feeding for further one week, similar procedure was performed. Further one week after feeding, whole blood was collected.

The obtained blood was allowed to stand at a temperature of 4 °C for 18 hours and then centrifuged at 3,000 r.p.m. for 15 minutes in a conventional manner to give anti-luciferase serum as the supernatant.

4. Synthesis of c-DNA

Synthesis of c-DNA was carried out using a kit manufactured by Amersham Co., Ltd.

Using 2 µg of m-RNA obtained as described above, synthesis of c-DNA was carried out in accordance with the methods described in Mol. Cell Biol., 2, 161 (1982) and Gene, 25, 263 (1983). As the result, 300 ng of double stranded c-DNA was obtained.

This c-DNA, 150 ng, was dissolved in 7 µl of TE buffer [10 mM Tris-hydrochloride buffer (pH 7.5)/1 mM EDTA]. To the solution were added, respectively, 11 µl of a mixture [280 mM sodium cacodylate (pH 6.8)-/60 mM Tris-hydrochloride buffer (pH 6.8)/2 mM cobalt chloride] and 3.8 µl of a tailing mixture [7.5 µl of 10 mM dithiothreitol/1 µl of 10 ng/ml poly(A)/2 µl of 5mM dCTP/110 µl of water]. Furthermore, 29 units of terminal transferase (manufactured by Boehringer Mannheim GmbH) was added to the mixture. After reacting at a temperature of 30 °C for 10 minutes, 2.4 µl of 0.25 M EDTA and 2.4 µl of 10% (W/V) sodium dodecylsulfate were added to the mixture to discontinue the reaction.

The solution in which the reaction had been discontinued was subjected to a treatment for removing protein using 25 µl of water-saturated phenol. Then, 25 µl of 4 M ammonium acetate and 100 µl of cold ethanol were added to the recovered aqueous phase, respectively. The mixture was allowed to stand at a temperature of - 70 °C for 15 minutes and centrifuged at 12,000 r.p.m. for 10 minutes to recover c-DNA. The c-DNA was dissolved in 10 µl of TE buffer to give a c-DNA solution.

As described above, 100 ng of the c-DNA with the deoxycytidine tail was obtained.

5. Production of recombinant plasmid pMCE10 DNA used in vector

Plasmid pKN305 DNA produced by the method described in T. Masuda et al., Agricultural Biological Chemistry, 50, 271-279 (1986) using plasmid pBR325 (manufactured by BRL Co.) and plasmid pBR322 DNA (manufactured by Takara Shuzo Co., Ltd.), and pMC1403-3 DNA (described in Japanese Patent Publication KOKAI 61-274683) were added by 1 µg each to 10 µl of a mixture [50 mM Tris-hydrochloride buffer (pH 7.5)/10 mM $MgCl_2$/100 mM NaCl/1 mM dithiothreitol]. Further, 2 units each of Hind III and Sal I (both manufactured by Takara Shuzo Co., Ltd.) were added to the mixture. By reacting at a temperature of 37°C for an hour, a cleavage treatment was effected. Extraction with phenol and precipitation with ethanol were conducted in a conventional manner to give precipitates. The precipitates were dissolved in 10 µl of ligation buffer [20 mM $MgCl_2$/66 mM Tris-hydrochloride buffer (pH 7.6)/1 mM ATP/15 mM dithiothreitol] to give a solution. Furthermore, 1 unit of T4 DNA ligase (manufactured by Takara Shuzo Co., Ltd.) was added thereto to perform ligation at a temperature of 20°C for 4 hours. Then, using this reaction solution, E. coli JM101 (ATCC 33876) was transformed according to the transformation method described in [J. Bacteriology, 119, 1072-1074 (1974)]. By examination of chemical resistance (ampicillin resistance and tetracycline sensitivity) and β-galactosidase activity, a transformant was obtained. Recombinant plasmid DNA contained in the strain was named pMCE10. E. coli JM101 strain containing this recombinant plasmid DNA pMCE10 DNA was cultured in medium composed of 1% (W/V) of trypton, 0.5% (W/V) of yeast extract and 0.5% (W/V) of NaCl at a temperature of 37°C for 16 to 24 hours. Twenty milliliters of the thus obtained culture solution of E. coli JM101 (pMCE10) was inoculated on 1 liter of the medium followed by shake culture at a temperature of 37°C for 3 hours. After the addition of 0.2 g of chloramphenicol, cultivation was conducted at the same temperature for further 20 hours to give a culture solution.

Next, the culture solution was centrifuged at 6,000 r.p.m. for 10 minutes in a conventional manner to give 2 g of wet cells. After the cells were suspended in 20 ml of 350 mM Tris-hydrochloride buffer (pH 8.0) containing 25% (W/V) sucrose, 10 mg of lysozyme, 8 ml of 0.25 M EDTA solution (pH 8.0) and 8 ml of 20% (W/V) sodium dodecylsulfate were added to the suspension, respectively. The mixture was kept at a temperature of 60°C for 30 minutes to give a lysate solution.

To the lysate solution was added 13 ml of 5 M NaCl solution. The mixture was treated at a temperature of 4°C for 16 hours and then centrifuged at 15,000 r.p.m. in a conventional manner to give an extract. The extract was subjected to the phenol extraction and the ethanol precipitation in a conventional manner to give precipitates.

Then, the precipitates were dried under reduced pressure in a conventional manner and dissolved in 10 mM Tris-hydrochloride buffer (pH 7.5) containing 1 mM EDTA. To the solution were further added 6 g of cesium chloride and 0.2 ml of ethydium bromide solution (10 mg/ml). The resulting mixture was subjected to an equilibrated density gradinent centrifugation treatment using a ultracentrifuging machine at 39,000 r.p.m. for 42 hours in a conventional manner thereby to isolate recombinant plasmid pMCE10 DNA. After ethydium bromide was removed using n-butanol, dialysis was performed to 10 mM Tris-hydrochloride buffer (pH 7.5) containing 1 mM EDTA to give 500 µg of purified recombinant plasmid pMCE10 DNA.

6. Production of vector DNA

The thus obtained recombinant plasmid pMCE10 DNA, 15 µg, was dissolved in 90 µl of TE buffer described in Item 4. After 10 µl of Med buffer [100 mM Tris-hydrochloride buffer (pH 7.5)/10 mM $MgCl_2$/10 mM dithiothreitol/500 mM NaCl] was added to the solution, 30 units of restriction enzyme Acc I (manufactured by Takara Shuzo Co., Ltd.) was further added to the mixture. A cleavage treatment was conducted at a temperature of 37°C for an hour to give the cleavage product. To the cleavage product was added 100 µl of water-saturated phenol, whereby protein was removed. Then, the aqueous phase was recovered and a 1/10-fold amount of 3 M sodium acetate (pH 7.5) and a 2-fold amount of cold ethanol were added to the aqueous phase. After allowing to stand at a temperature of -70°C for 15 minutes, the mixture was centrifuged at 12,000 r.p.m. for 10 minutes to recover DNA.

This DNA was dissolved in 10 µl of TE buffer and 15 µl of a mixture [280 mM sodium cacodylate (pH 6.8)/60 mM Tris-hydrochloride buffer (pH 6.8)/2 mM cobalt chloride] was added to the solution. Then, 5 µl of a tailing solution mixture (described in Item 4) (5 mM dGTP was used) was further added to the mixture. Furthermore, 5 units of terminal transferase (manufactured by Takara Shuzo Co., Ltd.) was added to react at a temperature of 37°C for 15 minutes. By post-treatment in a manner similar to the c-DNA tailing reaction described in Item 4, DNA with the deoxyguanosine tail at the Acc I site of recombinant plasmid pMCE10

DNA was produced.

On the other hand, DNA with the deoxyguanosine tail at the Pst I site of plasmid pUC19 DNA was also produced at the same time.

To a solution of 30 µg of plasmid pUC19 DNA (manufactured by Takara Shuzo Co., Ltd.) in 350 µl of TE buffer were added 40 µl of Med buffer and 120 units of restriction enzyme Pst I (manufactured by Takara Shuzo Co., Ltd.). After a cleavage treatment at a temperature of 37°C for an hour, DNA was recovered by the phenol treatment for removing protein and ethanol precipitation in a conventional manner.

The obtained DNA was dissolved in 35 µl of TE buffer. To the solution were added 50 µl of a mixture [280 mM sodium cacodylate (pH 6.8)/60 mM Tris-hydrochloride buffer (pH 6.8)/1 mM cobalt chloride], 19 µl of the tailing mixture (containing dGTP instead of dCTP) described in Item 4 and 60 units of terminal transferase (manufactured by Takara Shuzo Co., Ltd.). After reacting at a temperature of 37°C for 10 minutes, DNA was recovered by the phenol treatment for removing protein and ethanol precipitation in a conventional manner.

7. Annealing and transformation

The thus synthesized c-DNA, 15 ng and 200 ng of vector DNA were dissolved in 35 µl of annealing buffer [10 mM Tris-hydrochloride buffer (pH 7.5)/100 mM NaCl/1 mM EDTA]. The solution was allowed to stand at a temperature of 65°C for 2 minutes, at a temperature of 46°C for 2 hours, at a temperature of 37°C for an hour and at a temperature of 20°C for 18 hours thereby to anneal c-DNA and vector DNA.

Using the annealed DNA, E. coli DH1 strain (ATCC 33849) was transformed by the method of Hanahan [DNA Cloning, 1, 109-135 (1985)] to produce a c-DNA bank containing plasmid pUC19 DNA and recombinant plasmid pMCE10 DNA as vectors, respectively.

8. Survey of luciferase c-DNA

The Acc I site of recombinant plasmid pMCE10 DNA is present at a site which codes for E. coli B-galactosidase gene. Therefore, c-DNA incorporated into this site forms a fused protein with β-galactosidase. Furthermore, a promoter of β-galactosidase gene of the recombinant plasmid pMCE10 DNA has been converted into a promoter of E. coli tryptophan gene, as described above.

96 colonies of c-DNA having recombinant plasmid pMCE10 DNA as a vector were shake cultured in 10 ml of M9 Casamino acid medium [Molecular Cloning, 440-441, Cold Spring Harbor Laboratory (1982)] supplemented with thiamine (10 µg/ml) at a temperature of 37°C for 10 hours. After collecting the cells in a conventional manner, the cells were suspended in 200 µl of sample buffer for SDS-PAGE described in Item 2. The suspension was boiled at a temperature of 100°C for 5 minutes. This suspension, 40 µl, was subjected to electrophoresis in a conventional manner using 7.5% (W/V) polyacrylamide gel. After completion of the electrophoresis, the protein developed on the gel was transferred onto a nitrocellulose filter by the western blot method [Anal. Biochem., 112, 195 (1981)]. This nitrocellulose filter was stained with anti-luciferase serum using immune blot assay kit (manufactured by Biorad Co.). The method was performed in accordance with the instruction of Biorad Co.

That is, the nitrocellulose filter was shaken in 100 ml of blocking solution [a solution obtained by dissolving 3% (W/V) gelatin in TBS buffer [20 mM Tris-hydrochloride buffer /500 mM NaCl (pH 7.5)] at a temperature of 25°C for 30 minutes. Next, this nitrocellulose filter was transferred into 25 ml of primary antibody solution [solution obtained by dissolving 1% (W/V) gelatin in TBS buffer and diluting luciferase anti-serum with the resulting solution] and shaken at a temperature of 25°C for 90 minutes, which was then transferred into 100 ml Tween-20 washing solution [solution obtained by dissolving 0.05% (W/V) Tween-20 in TBS buffer] and shaken at a temperature of 25°C for 10 minutes. This procedure was repeated twice. Then, the thus obtained nitrocellulose filter was transferred into 60 ml of secondary antibody solution [solution obtained by dissolving anti-rabbit antibody labeled with horse raddish peroxidase (manufactured by Biorad Co.) with a solution of 1% (W/V) gelatin in TBS buffer to 3000-fold (V/V)]. After shaking at a temperature of 25°C for 60 minutes, the nitrocellulose filter was washed with 100 ml of Tween-20 washing solution. The procedure described above was repeated twice. The thus obtained nitrocellulose filter was transferred into 120 ml of color forming solution [solution obtained by mixing a solution of 60 mg of 4-chloro-1-naphthol in 20 ml of cold methanol and a solution of 60 µl of 30% (V/V) hydrogen peroxide aqueous solution in 100 ml of TBS buffer] to form a color at a temperature of 25°C for 10 minutes.

As such, similar procedures were performed on 4 groups, with 96 colonies per one group. In the two groups, protein band stained with luciferase anti-serum was recognized. Next, 96 colonies belonging to the two groups were divided into 8 groups with 12 colonies each and similar procedure was repeated. A protein

that reacted with anti-luciferase serum was noted in one group. Finally, with respect to the 12 colonies contained in this group, each colony was treated in a similar manner, whereby a protein-producing colony that reacted with luciferase anti-serum was identified. By the foregoing procedure, 2 colonies containing luciferase c-DNA were obtained. From the two colonies, plasmid DNA was produced by the method described in Item 5. The obtained recombinant plasmid DNAs were named pALf2B8 and PALF3A6, respectively.

9. Survey of large luciferase c-DNA - Production of c-DNA probe

In 330 µl of TE buffer was dissolved 100 µg of recombinant plasmid, pALf3A6 DNA. To the solution were added 40 µl of Low buffer [100 mM Tris-hydrochloride buffer (pH 7.5)/100 mM $MgCl_2$/10 mM dithiothreitol], 130 units of Pst I (manufactured by Takara Shuzo Co., Ltd.) and 120 units of Sac I (manufactured by Boehringer Mannheim GmbH) to effect cleavage at a temperature of 37 °C for 1.5 hours.

The whole amount of DNA was separated by electrophoresis using 0.7% (W/V) agarose gel. The agarose gel electrophoresis was carried out in accordance with the method of T. Maniatis et al., Molecular Cloning, pages 156-161, Cold Spring Harbor Laboratory (1984)]. DNA band containing luciferase c-DNA was excised and put in a dialysis tube. After 2 ml of TE buffer was supplemented, the dialysis tube was sealed and DNA was eluted from the gel into the buffer by electrophoresis. An equivalent volume of water-saturated phenol was added to this solution. After agitation, the aqueous phase was recovered and DNA was recovered by precipitation with ethanol in a conventional manner.

10 µg of the obtained DNA fragment was dissolved in TE buffer and 16 µl of Med buffer and 64 units of Sau 3 AI (manufactured by Takara Shuzo Co., Ltd.) were added to the solution. After reacting at a temperature of 37 °C for 2 hours, the whole amount was subjected to electrophoresis using 5% (W/V) polyacrylamide gel thereby to isolate DNA fragments. The polyacrylamide gel electrophoresis was carrried out in accordance with the method of A. Maxam [Methods in Enzymology, 65, 506 (1980)]. DNA fragment of 190 bp was isolated by the method as described above to give 1 µg of Sau3 AI luciferase c-DNA fragment.

Using [$\alpha$-$^{32}$P] dCTP (manufactured by Amersham Co.), 1 µg of this luciferase c-DNA was labeled according to the nick translation method. The nick translation method was performed using a kit manufactured by Takara Shuzo Co., Ltd. in accordance with the method described in J. Mol. Biol., 113, 237-251 (1977) and Molecular Cloning, pages 109-112, Cold Spring Harbor Laboratory (1982).

10. Survey of large luciferase c-DNA - Colony hybridization

Using as a probe the luciferase c-DNA fargment labelled with $^{32}$P produced by the method described above, c-DNA bank of the posterior portion of Photinus pyralis wherein recombinant plasmid pUC19 DNA was a vector was surveyed by colony hybridization [(Protein, Nucleic Acid and Enzyme, 26, 575-579 (1981)] to give colonies having luciferase c-DNA. Recombinant plasmid DNA possessed by one of the colonies was named pALf3 and plasmid DNA was produced by the method described in Item 5. E. coli containing the recombinant plasmid DNA was named E. coli DH 1 (pALf3). The transformant has been deposited as ATCC 67462.

The recombinant plasmid pALf3 DNA described above was subjected to single digestion and double digestion using Xba I, Hind III, BamH I, Eco RI and Pst I (all manufactured by Takara Shuzo Co., Ltd.). The obtained DNA fragments were analyzed by agarose gel electrophoresis on mobility pattern. By comparing the obtained mobility pattern with standard mobility pattern of DNA fragment obtained by digesting λ phage DNA (manufactured by Takara Shuzo Co., Ltd.) with Hind III, the size of the c-DNA inserted in pALf3 was turned out to be 1,700 bp. A restriction enzyme map of the plasmid described above is shown in Fig. 3.

11. Preparation of m-RNA of Luciola cruciata

Ten grams of living Luciola cruciata (GENJI firefly, purchased from Seibu Department Store) were put in a ultra-low temperature freezer box and frozen. Each posterior portion was cut off with scissors. To 2 g of the obtained posterior portion was added 18 ml of guanidine isothiocyanate solution. According to the method described in Item 1, 1.1 mg of RNA was prepared. In accordance with the method described in Item 1, 1.1 mg of this RNA was subjected to column chromatography of oligo (dT)-cellulose to obtain 30 µg of m-RNA for the posterior portion of Luciola cruciata.

12. Production of c-DNA bank of Luciola cruciata posterior portion

Synthesis of c-DNA was performed using a kit purchased from Amersham Co. in accordance with the method indicated by Amersham Co. which is described in Mol. Cell Biol., 2, 161 (1982) and Gene, 25, 263 (1983).

From 2 µg of the Luciola cruciata posterior portion RNA, 0.9 µg of double stranded c-DNA was synthesized. Using the method described in Item 4, a tail of polydeoxycytidine was added to 0.3 µg of this c-DNA.

This c-DNA, 20 ng, and 500 ng of pUC19 plasmid produced in Item 6, wherein a polyguanosine tail had been added to the Pst I site thereof, were annealed in accordance with the method described in Item 7. E. coli DH 1 strain (ATCC 33849) was transformed by annealed DNA by the method of Hanahan [DNA Cloning, 1, 109-135 (1985)] thereby to produce c-DNA bank of Luciola cruciata tail.

13. Survey of luciferase c-DNA derived from Luciola cruciata

In 90 µl of TE buffer was dissolved 10 µg of recombinant plasmid pALf3 DNA obtained in Item 10 and, 10 µl of Med buffer, 25 units of restriction enzyme Eco RI and 25 units of restriction enzyme Cla I (both manufactured by Takara Shuzo Co., Ltd.) were added to the solution. The reaction was performed at a temperature of 37 °C for 2 hours to cleave DNA. From the cleaved recombinant plasmid pALf3 DNA, 800 bp of Eco RI/Cla I DNA fragment containing luciferase c-DNA derived from Photinus pyralis (American firefly) was isolated in accordance with the method described in Item 9 using agarose gel electrophoresis. Thus, 1 µg of Eco RI/Cla I DNA fragment was obtained. Using [$\alpha$-$^{32}$P] dCTP (manufactured by Amersham Co.), 1 µg of this DNA was labelled with $^{32}$P in accordance with the nick translation method described in Item 9. Using as a probe the Eco RI/Cla I DNA fragment labeled with $^{32}$P, c-DNA bank of the Luciola cruciata posterior portion was surveyed by the colony hybridization described in Item 10 thereby to select E. coli having luciferase c-DNA derived from Luciola cruciata. Several colonies of E. coli capable of hybridizing with the probe were obtained. Recombinant plasmid DNA possessed by one of these colonies was named pGLf1. The recombinant plasmid DNA was isolated in accordance with the method described in Item 5.

The recombinant plasmid pGLf1 DNA described above was subjected to single digestion and double digestion using Hpa I, Hind III, Eco RV, Dra I, Af1 II, Hinc II, Pst I (all manufactured by Takara Shuzo Co., Ltd.) and Ssp I (manufactured by New England Biolab Co.). The obtained DNA fragments were analyzed by agarose gel electrophoresis on mobility pattern. By comparing the obtained mobility pattern with standard mobility pattern of DNA fragment obtained by digesting λ phage DNA (manufactured by Takara Shuzo Co., Ltd.) with Hind III, the size of the c-DNA inserted in pGLf1 was turned out to be 2,000 bp. A restriction enzyme map of the plasmid described above is shown in Fig. 4.

14. Preparation of m-RNA of Luciola lateralis

Five grams of living Luciola lateralis (HEIKE firefly, purchased from Kawahara Choju Trading Co., Ltd.) were put in a ultra-low temperature freezer box and frozen. Each posterior portion was cut off with scissors. To 1 g of the obtained posterior portion was added 18 ml of guanidine isothiocyanate solution. Following the method described in Item 1, 340 µg of RNA was prepared. In accordance with the method described in Item 1, 340 µg of the RNA was subjected to column chromatography of oligo(dT)-cellulose to obtain 6 µg of m-RNA from the posterior portion of Luciola lateralis.

15. Production of c-DNA bank of Luciola lateralis posterior portion

Synthesis of c-DNA was performed using a kit purchased from Amersham Co.

Using 2.0 µg of m-RNA obtained as above, c-DNA was synthesized in accordance with the method indicated by Amersham Co. which is described in Mol. Cell Biol., 2, 161 (1982) and Gene, 25, 263 (1983). As the result, 250 ng of double stranded c-DNA was obtained.

The thus obtained c-DNA, 250 ng, was subjected to methylation at the restriction enzyme Eco RI site using c-DNA cloning kit manufactured by Amersham Co. as instructed by Amersham Co. Furthermore, Eco RI linker was adhered to the both termini of the c-DNA.

After 1 µl of Med buffer [100 mM Tris-hydrochloride buffer (pH 7.5)/100 mM $MgCl_2$/10 mM dithiothreitol/500 mM NaCl] was added to a solution of 100 ng of plasmid pUC119 DNA (manufactured by Takara Shuzo Co., Ltd.) in 8 µl of water, 10 units of restriction enzyme Eco RI (manufactured by Takara Shuzo Co., Ltd.) was further added to the mixture. A cleavage treatment was conducted at a temperature of

37°C for an hour.

Subsequently, 1 μl of 1M Tris-hydrochloride buffer (pH 8.0) and 0.3 unit (1 μl) of alkaline phosphatase (manufactured by Takara Shuzo Co., Ltd.) were added to the cleavage product and the mixture was subjected to enzymatic reaction at a temperature of 65°C for an hour to effect dephosphorylation of the termini of the cleavage product. After 12 μl of water-saturated phenol was added to the dephosphorylated product to remove protein, 1 μl of 3M sodium acetate (pH 5.8) and 26 μl of cold ethanol were added to the recovered aqueous phase, respectively. The mixture was allowed to stand at a temperature of -70°C for 15 minutes. By centrifuging treatment at 12,000 r.p.m. for 5 minutes with a trace centrifuging machine (manufactured by TOMI SEIKO K.K., MRX-150) to recover DNA.

The thus obtained DNA was cleaved with restriction enzyme Eco RI and its termini were dephospholyated. The resulting plasmid vector pUC119 DNA, 100 ng, was mixed with 250 ng of c-DNA produced in Item 15. After the mixutre was suspended in 8 μl of water, 1 μl of ligation buffer [200 mM $MgCl_2$/660 mM Tris-hydrochloride buffer (pH 7.6)/10 mM ATP/150 mM dithiothreitol] was added to the resulting mixture. Furthermore, 1 unit of T4 DNA ligase (manufactured by Takara Shuzo Co., Ltd.) was added thereto and the mixture was allowed to stand at a temperature of 16°C for 16 hours, whereby ligation of the plasmid vector and c-DNA was performed to give the reaction product.

Using this reaction product, E. coli DH1 (ATCC 33849) strain was transformed by the method of Hanahan [DNA Cloning, 1, 109-135 (1985)] to produce a c-DNA bank derived from the posterior portion of Luciola lateralis containing plasmid pUC119 DNA as a vector.

16. Survey of luciferase c-DNA derived from Luciola lateralis

In 90 μl of TE buffer was dissolved 10 μg of recombinant plasmid pGLf1 DNA obtained in Item 13 and, 10 μl of Med buffer, 25 units of restriction enzyme Pst I (manufactured by Takara Shuzo Co., Ltd.) was added to the solution. The reaction was performed at a temperature of 37°C for 2 hours to cleave DNA. From the cleaved recombinant plasmid pGLf1 DNA, 2,000 bp of Pst I DNA fragment containing Luciola cruciata-derived luciferase c-DNA portion was isolated in accordance with the method described in Item 9 using agarose gel electrophoresis. Thus, 1 μg of Pst I DNA fragment was obtained. Using [$\alpha$-$^{32}P$] dCTP (manufactured by Amersham Co.), 1 μg of this DNA was labelled with $^{32}P$ in accordance with the nick translation method described in Item 9. Using as a probe the Pst I DNA fragment labeled with $^{32}P$, c-DNA bank of the Luciola lateralis posterior portion was surveyed by the colony hybridization described in Item 10 thereby to select E. coli bearing luciferase c-DNA derived from Luciola lateralis. Several colonies of E. coli capable of hybridizing with the probe were obtained. Plasmid DNA possessed by one of these colonies was named pHLf7. The recombinant plasmid DNA was isolated in accordance with the method described in Item 5.

The thus obtained E. coli DH1 (pHLf7) has been deposited in the Fermentation Research Institute, the Agency of Industrial Science and Technology, Japan, under the Budapest Treaty with the accession number FERM BP-1917.

The recombinant plasmid pHLf7 DNA described above was subjected to single digestion and double digestion using Hpa I, Eco RV, Apa I, Hind III and Eco RI (all manufactured by Takara Shuzo Co., Ltd.) and Ssp I (manufactured by New England Biolab Co.). The obtained DNA fragments were analyzed by agarose gel electrophoresis on mobility pattern. By comparing the obtained mobility pattern with standard mobility pattern of DNA fragment obtained by digesting λ phage DNA (manufactured by Takara Shuzo Co., Ltd.) with Hind III, the size of the gene encoding Luciola lateralis-derived luciferase was turned out to be 2,000 bp. A restriction enzyme map of the plasmid described above is shown in Fig. 5.

To a solution of 10 μg of recombinant plasmid pHLf7 DNA in 45 μl of TE buffer were added 5 μl of Med buffer and 2 units of restriction enzyme Eco RI (manufactured by Takara Shuzo Co., Ltd.), respectively. The mixture was reacted at a temperature of 37°C for 2 hours to give a partial digestion product of DNA.

Then, the partial digestion product was subjected to agarose gel electrophoresis described in Item 9 and 1 μg of Eco RI fragment of 2,000 bp containing all the gene encoding Luciola lateralis-derived luciferase was isolated.

17. Cultivation of E. coli DH1 (pHLf7) (FERM BP-1917) and production of crude enzyme solution

E. coli DH1 (pHLf7) (FERM BP-1917) was shake cultured in 3 ml of LB-amp medium [1% (W/V) bactotrypton, 0.5% (W/V) yeast extract, 0.5% (W/V) NaCl and ampicillin (50 μg/ml)] at a temperature of 37°C for 18 hours. This culture solution, 0.5 ml, was inoculated on 10 μl of the aforesaid LB-amp medium

and 1 mM isopropyl-β-D-thiogalactoside was added thereto. After shake culture at a temperature of 37°C for 4 hours, the culture was subjected to a centrifuging operation at 8,000 r.p.m. for 10 minutes to give 20 mg of wet cells.

The recovered cells were suspended in 0.9 ml of a buffer composed of 0.1 M $KH_2PO_4$ (pH 7.8), 2 mM EDTA, 1 mM dithiothreitol and 0.2 mg/ml protamine sulfate. Further 100 µl of 10 mg/ml lysozyme solution was supplemented to the suspension. The mixture was allowed to stand in ice for 15 minutes. Next, the suspension was frozen in methanol-dry ice bath and then allowed to stand at a temperature of 25°C to completely thaw. Further by performing a centrifuging operation at 12,000 r.p.m. for 5 minutes, 1 ml of crude enzyme solution was obtained as the supernatant.

The luciferase activity in the thus obtained crude enzyme solution was performed by the method described below. The results are shown in Table 1 below.

The measurement of luciferase activity in the crude enzyme solution obtained was performed by counting the number of photons generated in accordance with the method of Kricka [Archives of Biochemistry and Biophysics, 217, 674 (1982)].

That is, 260 µl of 25 mM glycylglycine buffer (pH 7.8), 16 µl of 0.1 M magnesium sulfate and 24 µl of 1 mM luciferine (manufactured by Sigma Inc.) and 10 µl of the crude enzyme solution were mixed. Then 100 µl of 20 mM ATP was added to the mixture. The number of photons generated was integrated for 20 seconds. The integrated values are shown in Table 1 below. For purpose of comparison, a luciferase activity was measured also with plasmid pUC119 DNA-bearing E. coli DH1 strain [E. coli DH1 (pUC119)]. The results are also shown in Table 1 below.

Table 1

| Item / Sample | Number of Photon/ml Culture Solution |
|---|---|
| E. coli DH1 (pHLf7) (invention) | $6.2 \times 10^6$ |
| E. coli DH1 (pUC119) (control) | $1.0 \times 10^4$ |

As is clear from the table above, it is noted that the count of photons increased in E. coli DH1 (pHLf7) bearing the recombinant plasmid pHLf7 containing the luciferase gene of this invention as compared to the control and therefore, luciferase is produced in the cells of E. coli used in this invention.

18. Analysis of nucleotide sequence of luciferase c-DNA derived from Luciola lateralis

Recombinant plasmid pHLf7 DNA, 10 µg, was cleaved with restriction enzyme Eco RI (manufactured by Takara Shuzo Co., Ltd.) to give 2.0 µg of 1.7 Kb DNA fragment and 0.5 µg of 0.3 Kb DNA fragment, containing luciferase c-DNA. These DNA fragments were subcloned at the Eco RI site of plasmid pUC118 DNA (manufactured by Takara Shuzo Co., Ltd.) to give 4 plasmids, pHLf11, pHLf12, pHLf13 and pHLf14, based on differences in kind of the inserted fragments (1.7 Kb and 0.3 Kb) and in orientation of the insertion (the 1.7 Kb fragment was subcloned to pHLf11 and pHLf12, and the 0.3 Kb fragment was subcloned to pHLf13 and pHLf14).

Cleavage treatment of the recombinant plasmids pHLf7 DNA and plasmid pUC118 DNA with Eco RI (method described in Item 6), isolation of the luciferase c-DNA fragment using agarose gel electrophoresis (method described in Item 9), ligation of plasmid pUC119 DNA and the luciferase c-DNA fragment (method described in Item 5), transformation of E. coli JM101 strain (ATCC 33876) using ligation reaction liquid (method described in Item 5) and production of recombinant plasmids pHLf11, pHLf12, pHLf13 and pHLf14 (method described in Item 5) followed the methods described within parentheses.

Next, using the recombinant plasmid DNAs pHLf11, pHLf12, pHLf13 and pHLf14, plasmid DNAs wherein various deletions were introduced into the luciferase c-DNA were produced using a deletion kit for

killosequence (manufactured by Takara Shuzo Co., Ltd.) in accordance with the method of Henikoff [Gene, 28, 351-359 (1984)]. These plasmid DNAs were introduced into E. coli JM101 strain (ATCC 33876) by the method described in Item 5. By infecting the thus obtained E. coli with helper phage M13K07 (manufactured by Takara Shuzo Co., Ltd.), single strand DNA was produced in accordance with the method of Messing [Methods in Enzymology, 101, 20-78 (1983)]. Sequencing with the obtained single strand DNA was carried out by the method of Messing described above, using M13 sequencing kit (manufactured by Takara Shuzo Co., Ltd.). Gel electrophoresis for analyzing a nucleotide sequence was carried out using 8% (W/V) polyacrylamide gel (manufactured by Fuji Photo Film Co., Ltd.). The nucleotide sequence of the Luciola lateralis-derived luciferase-coding c-DNA obtained is shown in Fig. 6. Fig. 7 shows an amino acid sequence of polypeptide translated from the c-DNA and Fig. 8 shows a sequence corresponding to c-DNA in the amino acid sequence.

## Claims

**1.** A Luciola lateralis-derived luciferase gene defined by a restriction enzyme map described below:

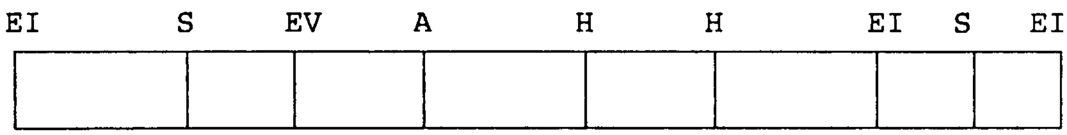


wherein EI represents Eco RI, S represents Ssp I, EV represents Eco RV, A represents Apa I and H represents Hpa I.

**2.** A luciferase gene according to claim 1, which encodes an amino acid sequence shown below:

| | | | | | | | | | |
|---|---|---|---|---|---|---|---|---|---|
| | | | | | | | | | 10 |
| Met | Glu | Asn | Met | Glu | Asn | Asp | Glu | Asn | Ile |
| | | | | | | | | | 20 |
| Val | Tyr | Gly | Pro | Glu | Pro | Phe | Tyr | Pro | Ile |
| | | | | | | | | | 30 |
| Glu | Glu | Gly | Ser | Ala | Gly | Ala | Gln | Leu | Arg |
| | | | | | | | | | 40 |
| Lys | Tyr | Met | Asp | Arg | Tyr | Ala | Lys | Leu | Gly |
| | | | | | | | | | 50 |
| Ala | Ile | Ala | Phe | Thr | Asn | Ala | Leu | Thr | Gly |
| | | | | | | | | | 60 |
| Val | Asp | Tyr | Thr | Tyr | Ala | Glu | Tyr | Leu | Glu |
| | | | | | | | | | 70 |
| Lys | Ser | Cys | Cys | Leu | Gly | Glu | Ala | Leu | Lys |
| | | | | | | | | | 80 |
| Asn | Tyr | Gly | Leu | Val | Val | Asp | Gly | Arg | Ile |
| | | | | | | | | | 90 |
| Ala | Leu | Cys | Ser | Glu | Asn | Cys | Glu | Glu | Phe |
| | | | | | | | | | 100 |
| Phe | Ile | Pro | Val | Leu | Ala | Gly | Leu | Phe | Ile |
| | | | | | | | | | 110 |
| Gly | Val | Gly | Val | Ala | Pro | Thr | Asn | Glu | Ile |
| | | | | | | | | | 120 |
| Tyr | Thr | Leu | Arg | Glu | Leu | Val | His | Ser | Leu |
| | | | | | | | | | 130 |
| Gly | Ile | Ser | Lys | Pro | Thr | Ile | Val | Phe | Ser |
| | | | | | | | | | 140 |
| Ser | Lys | Lys | Gly | Leu | Asp | Lys | Val | Ile | Thr |
| | | | | | | | | | 150 |
| Val | Gln | Lys | Thr | Val | Thr | Ala | Ile | Lys | Thr |
| | | | | | | | | | 160 |
| Ile | Val | Ile | Leu | Asp | Ser | Lys | Val | Asp | Tyr |
| | | | | | | | | | 170 |
| Arg | Gly | Tyr | Gln | Ser | Met | Asp | Asn | Phe | Ile |
| | | | | | | | | | 180 |
| Lys | Lys | Asn | Thr | Pro | Gln | Gly | Phe | Lys | Gly |
| | | | | | | | | | 190 |
| Ser | Ser | Phe | Lys | Thr | Val | Glu | Val | Asn | Arg |

200
Lys Glu Gln Val Ala Leu Ile Met Asn Ser
210
Ser Gly Ser Thr Gly Leu Pro Lys Gly Val
220
Gln Leu Thr His Glu Asn Ala Val Thr Arg
230
Phe Ser His Ala Arg Asp Pro Ile Tyr Gly
240
Asn Gln Val Ser Pro Gly Thr Ala Ile Leu
250
Thr Val Val Pro Phe His His Gly Phe Gly
260
Met Phe Thr Thr Leu Gly Tyr Leu Thr Cys
270
Gly Phe Arg Ile Val Met Leu Thr Lys Phe
280
Asp Glu Glu Thr Phe Leu Lys Thr Leu Gln
290
Asp Tyr Lys Cys Ser Ser Val Ile Leu Val
300
Pro Thr Leu Phe Ala Ile Leu Asn Arg Ser
310
Glu Leu Leu Asp Lys Tyr Asp Leu Ser Asn
320
Leu Val Glu Ile Ala Ser Gly Gly Ala Pro
330
Leu Ser Lys Glu Ile Gly Glu Ala Val Ala
340
Arg Arg Phe Asn Leu Pro Gly Val Arg Gln
350
Gly Tyr Gly Leu Thr Glu Thr Thr Ser Ala
360
Ile Ile Ile Thr Pro Glu Gly Asp Asp Lys
370
Pro Gly Ala Ser Gly Lys Val Val Pro Leu
380
Phe Lys Ala Lys Val Ile Asp Leu Asp Thr

| | | | | | | | | | |
|---|---|---|---|---|---|---|---|---|---|
| | | | | | | | | | 390 |
| Lys | Lys | Thr | Leu | Gly | Pro | Asn | Arg | Arg | Gly |
| | | | | | | | | | 400 |
| Glu | Val | Cys | Val | Lys | Gly | Pro | Met | Leu | Met |
| | | | | | | | | | 410 |
| Lys | Gly | Tyr | Val | Asp | Asn | Pro | Glu | Ala | Thr |
| | | | | | | | | | 420 |
| Arg | Glu | Ile | Ile | Asp | Glu | Glu | Gly | Trp | Leu |
| | | | | | | | | | 430 |
| His | Thr | Gly | Asp | Ile | Gly | Tyr | Tyr | Asp | Glu |
| | | | | | | | | | 440 |
| Glu | Lys | His | Phe | Phe | Ile | Val | Asp | Arg | Leu |
| | | | | | | | | | 450 |
| Lys | Ser | Leu | Ile | Lys | Tyr | Lys | Gly | Tyr | Gln |
| | | | | | | | | | 460 |
| Val | Pro | Pro | Ala | Glu | Leu | Glu | Ser | Val | Leu |
| | | | | | | | | | 470 |
| Leu | Gln | His | Pro | Asn | Ile | Phe | Asp | Ala | Gly |
| | | | | | | | | | 480 |
| Val | Ala | Gly | Val | Pro | Asp | Pro | Ile | Ala | Gly |
| | | | | | | | | | 490 |
| Glu | Leu | Pro | Gly | Ala | Val | Val | Val | Leu | Glu |
| | | | | | | | | | 500 |
| Lys | Gly | Lys | Ser | Met | Thr | Glu | Lys | Glu | Val |
| | | | | | | | | | 510 |
| Met | Asp | Tyr | Val | Ala | Ser | Gln | Val | Ser | Asn |
| | | | | | | | | | 520 |
| Ala | Lys | Arg | Leu | Arg | Gly | Gly | Val | Arg | Phe |
| | | | | | | | | | 530 |
| Val | Asp | Glu | Val | Pro | Lys | Gly | Leu | Thr | Gly |
| | | | | | | | | | 540 |
| Lys | Ile | Asp | Gly | Lys | Ala | Ile | Arg | Glu | Ile |
| Leu | Lys | Lys | Pro | Val | Ala | Lys | Met | | |

3. A luciferase gene according to Claim 1 or 2 which is represented by a nucleotide sequence shown below.

```
                                            30
ATG GAA AAC ATG GAG AAC GAT GAA AAT ATT
                                            60
GTG TAT GGT CCT GAA CCA TTT TAC CCT ATT
                                            90
GAA GAG GGA TCT GCT GGA GCA CAA TTG CGC
                                           120
AAG TAT ATG GAT CGA TAT GCA AAA CTT GGA
                                           150
GCA ATT GCT TTT ACT AAC GCA CTT ACC GGT
                                           180
GTC GAT TAT ACG TAC GCC GAA TAC TTA GAA
                                           210
AAA TCA TGC TGT CTA GGA GAG GCT TTA AAG
                                           240
AAT TAT GGT TTG GTT GTT GAT GGA AGA ATT
                                           270
GCG TTA TGC AGT GAA AAC TGT GAA GAA TTC
                                           300
TTT ATT CCT GTA TTA GCC GGT TTA TTT ATA
                                           330
GGT GTC GGT GTG GCT CCA ACT AAT GAG ATT
                                           360
TAC ACT CTA CGT GAA TTG GTT CAC AGT TTA
                                           390
GGC ATC TCT AAG CCA ACA ATT GTA TTT AGT
                                           420
TCT AAA AAA GGA TTA GAT AAA GTT ATA ACT
                                           450
GTA CAA AAA ACG GTA ACT GCT ATT AAA ACC
                                           480
ATT GTT ATA TTG GAC AGC AAA GTG GAT TAT
                                           510
AGA GGT TAT CAA TCC ATG GAC AAC TTT ATT
                                           540
AAA AAA AAC ACT CCA CAA GGT TTC AAA GGA
                                           570
TCA AGT TTT AAA ACT GTA GAA GTT AAC CGC
```

600
AAA GAA CAA GTT GCT CTT ATA ATG AAC TCT
630
TCG GGT TCA ACC GGT TTG CCA AAA GGT GTG
660
CAA CTT ACT CAT GAA AAT GCA GTC ACT AGA
690
TTT TCT CAC GCT AGA GAT CCA ATT TAT GGA
720
AAC CAA GTT TCA CCA GGC ACG GCT ATT TTA
750
ACT GTA GTA CCA TTC CAT CAT GGT TTT GGT
780
ATG TTT ACT ACT TTA GGC TAT CTA ACT TGT
810
GGT TTT CGT ATT GTC ATG TTA ACG AAA TTT
840
GAC GAA GAG ACT TTT TTA AAA ACA CTG CAA
870
GAT TAC AAA TGT TCA AGC GTT ATT CTT GTA
900
CCG ACT TTG TTT GCA ATT CTT AAT AGA AGT
930
GAA TTA CTC GAT AAA TAT GAT TTA TCA AAT
960
TTA GTT GAA ATT GCA TCT GGC GGA GCA CCT
990
TTA TCT AAA GAA ATT GGT GAA GCT GTT GCT
1020
AGA CGT TTT AAT TTA CCG GGT GTT CGT CAA
1050
GGC TAT GGT TTA ACA GAA ACA ACC TCT GCA
1080
ATT ATT ATC ACA CCG GAA GGC GAT GAT AAA
1110
CCA GGT GCT TCT GGC AAA GTT GTG CCA TTA
1140
TTT AAA GCA AAA GTT ATC GAT CTT GAT ACT

```
                                             1170
AAA AAA ACT TTG GGC CCG AAC AGA CGT GGA
                                             1200
GAA GTT TGT GTA AAG GGT CCT ATG CTT ATG
                                             1230
AAA GGT TAT GTA GAT AAT CCA GAA GCA ACA
                                             1260
AGA GAA ATC ATA GAT GAA GAA GGT TGG TTG
                                             1290
CAC ACA GGA GAT ATT GGG TAT TAC GAT GAA
                                             1320
GAA AAA CAT TTC TTT ATC GTG GAT CGT TTG
                                             1350
AAG TCT TTA ATC AAA TAC AAA GGA TAT CAA
                                             1380
GTA CCA CCT GCT GAA TTA GAA TCT GTT CTT
                                             1410
TTG CAA CAT CCA AAT ATT TTT GAT GCC GGC
                                             1440
GTT GCT GGC GTT CCA GAT CCT ATA GCT GGT
                                             1470
GAG CTT CCG GGA GCT GTT GTT GTA CTT GAA
                                             1500
AAA GGA AAA TCT ATG ACT GAA AAA GAA GTA
                                             1530
ATG GAT TAC GTT GCT AGT CAA GTT TCA AAT
                                             1560
GCA AAA CGT TTG CGT GGT GGT GTC CGT TTT
                                             1590
GTG GAC GAA GTA CCT AAA GGT CTC ACT GGT
                                             1620
AAA ATT GAC GGT AAA GCA ATT AGA GAA ATA

CTG AAG AAA CCA GTT GCT AAG ATG
```

4. A novel recombinant DNA comprising a vector DNA into which a gene coding for Luciola lateralis-derived luciferase according to claims 1-3 is inserted.

5. A novel recombinant DNA according to claim 4, wherein said vector DNA is plasmid pUC119.

6. A method for producing a luciferase which comprises culturing in a medium a microorganism belonging to the genus Escherichia and bearing a recombinant DNA according to claim 4 obtained by inserting a gene coding for Luciola lateralis-derived luciferase into a vector DNA and collecting said luciferase from the culture.

7. A method for producing a luciferase according to claim 6, wherein said vector DNA is plasmid pUC119.

## Patentansprüche

1. Ein von Luciola lateralis abgeleitetes Luciferase-Gen, definiert durch die nachfolgend angegebene Restriktionsenzymkarte:

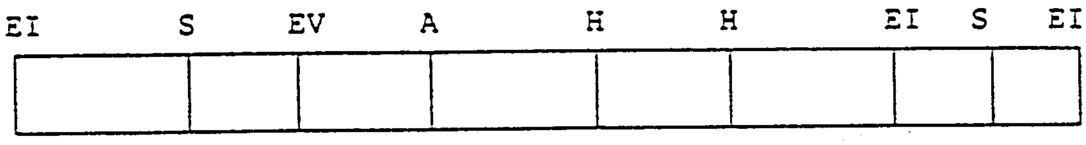


worin EI Eco RI bedeutet, S Ssp I bedeutet, EV Eco RV bedeutet, A Apa I bedeutet und H Hpa I bedeutet.

2. Luciferase-Gen nach Anspruch 1, dadurch gekennzeichnet, daß es die nachfolgend angegebene Aminosäuresequenz codiert:

```
                                              10
Met Glu Asn Met Glu Asn Asp Glu Asn Ile
                                              20
Val Tyr Gly Pro Glu Pro Phe Tyr Pro Ile
                                              30
Glu Glu Gly Ser Ala Gly Ala Gln Leu Arg
                                              40
Lys Tyr Met Asp Arg Tyr Ala Lys Leu Gly
                                              50
Ala Ile Ala Phe Thr Asn Ala Leu Thr Gly
                                              60
Val Asp Tyr Thr Tyr Ala Glu Tyr Leu Glu
                                              70
Lys Ser Cys Cys Leu Gly Glu Ala Leu Lys
```

80
Asn Tyr Gly Leu Val Val Asp Gly Arg Ile
90
Ala Leu Cys Ser Glu Asn Cys Glu Glu Phe
100
Phe Ile Pro Val Leu Ala Gly Leu Phe Ile
110
Gly Val Gly Val Ala Pro Thr Asn Glu Ile
120
Tyr Thr Leu Arg Glu Leu Val His Ser Leu
130
Gly Ile Ser Lys Pro Thr Ile Val Phe Ser
140
Ser Lys Lys Gly Leu Asp Lys Val Ile Thr
150
Val Gln Lys Thr Val Thr Ala Ile Lys Thr
160
Ile Val Ile Leu Asp Ser Lys Val Asp Tyr
170
Arg Gly Tyr Gln Ser Met Asp Asn Phe Ile
180
Lys Lys Asn Thr Pro Gln Gly Phe Lys Gly
190
Ser Ser Phe Lys Thr Val Glu Val Asn Arg
200
Lys Glu Gln Val Ala Leu Ile Met Asn Ser
210
Ser Gly Ser Thr Gly Leu Pro Lys Gly Val
220
Gln Leu Thr His Glu Asn Ala Val Thr Arg
230
Phe Ser His Ala Arg Asp Pro Ile Tyr Gly
240
Asn Gln Val Ser Pro Gly Thr Ala Ile Leu
250
Thr Val Val Pro Phe His His Gly Phe Gly
260
Met Phe Thr Thr Leu Gly Tyr Leu Thr Cys
270
Gly Phe Arg Ile Val Met Leu Thr Lys Phe
280
Asp Glu Glu Thr Phe Leu Lys Thr Leu Gln

290
Asp Tyr Lys Cys Ser Ser Val Ile Leu Val
300
Pro Thr Leu Phe Ala Ile Leu Asn Arg Ser
310
Glu Leu Leu Asp Lys Tyr Asp Leu Ser Asn
320
Leu Val Glu Ile Ala Ser Gly Gly Ala Pro
330
Leu Ser Lys Glu Ile Gly Glu Ala Val Ala
340
Arg Arg Phe Asn Leu Pro Gly Val Arg Gln
350
Gly Tyr Gly Leu Thr Glu Thr Thr Ser Ala
360
Ile Ile Ile Thr Pro Glu Gly Asp Asp Lys
370
Pro Gly Ala Ser Gly Lys Val Val Pro Leu
380
Phe Lys Ala Lys Val Ile Asp Leu Asp Thr
390
Lys Lys Thr Leu Gly Pro Asn Arg Arg Gly
400
Glu Val Cys Val Lys Gly Pro Met Leu Met
410
Lys Gly Tyr Val Asp Asn Pro Glu Ala Thr
420
Arg Glu Ile Ile Asp Glu Glu Gly Trp Leu
430
His Thr Gly Asp Ile Gly Tyr Tyr Asp Glu
440
Glu Lys His Phe Phe Ile Val Asp Arg Leu
450
Lys Ser Leu Ile Lys Tyr Lys Gly Tyr Gln
460
Val Pro Pro Ala Glu Leu Glu Ser Val Leu
470
Leu Gln His Pro Asn Ile Phe Asp Ala Gly
480
Val Ala Gly Val Pro Asp Pro Ile Ala Gly
490
Glu Leu Pro Gly Ala Val Val Val Leu Glu
500
Lys Gly Lys Ser Met Thr Glu Lys Glu Val

```
                                             510
Met Asp Tyr Val Ala Ser Gln Val Ser Asn
                                             520
Ala Lys Arg Leu Arg Gly Gly Val Arg Phe
                                             530
Val Asp Glu Val Pro Lys Gly Leu Thr Gly
                                             540
Lys Ile Asp Gly Lys Ala Ile Arg Glu Ile

Leu Lys Lys Pro Val Ala Lys Met
```

3. Luciferase-Gen gemäß Anspruch 1 oder 2, dadurch gekennzeichnet, daß es durch die nachfolgend angegebene Nucleotidsequenz dargestellt wird.

```
                                              30
ATG GAA AAC ATG GAG AAC GAT GAA AAT ATT
                                              60
GTG TAT GGT CCT GAA CCA TTT TAC CCT ATT
                                              90
GAA GAG GGA TCT GCT GGA GCA CAA TTG CGC
                                             120
AAG TAT ATG GAT CGA TAT GCA AAA CTT GGA
                                             150
GCA ATT GCT TTT ACT AAC GCA CTT ACC GGT
                                             180
GTC GAT TAT ACG TAC GCC GAA TAC TTA GAA
                                             210
AAA TCA TGC TGT CTA GGA GAG GCT TTA AAG
                                             240
AAT TAT GGT TTG GTT GTT GAT GGA AGA ATT
                                             270
GCG TTA TGC AGT GAA AAC TGT GAA GAA TTC
                                             300
TTT ATT CCT GTA TTA GCC GGT TTA TTT ATA
                                             330
GGT GTC GGT GTG GCT CCA ACT AAT GAG ATT
```

TAC ACT CTA CGT GAA TTG GTT CAC AGT TTA 360
GGC ATC TCT AAG CCA ACA ATT GTA TTT AGT 390
TCT AAA AAA GGA TTA GAT AAA GTT ATA ACT 420
GTA CAA AAA ACG GTA ACT GCT ATT AAA ACC 450
ATT GTT ATA TTG GAC AGC AAA GTG GAT TAT 480
AGA GGT TAT CAA TCC ATG GAC AAC TTT ATT 510
AAA AAA AAC ACT CCA CAA GGT TTC AAA GGA 540
TCA AGT TTT AAA ACT GTA GAA GTT AAC CGC 570
AAA GAA CAA GTT GCT CTT ATA ATG AAC TCT 600
TCG GGT TCA ACC GGT TTG CCA AAA GGT GTG 630
CAA CTT ACT CAT GAA AAT GCA GTC ACT AGA 660
TTT TCT CAC GCT AGA GAT CCA ATT TAT GGA 690
AAC CAA GTT TCA CCA GGC ACG GCT ATT TTA 720
ACT GTA GTA CCA TTC CAT CAT GGT TTT GGT 750
ATG TTT ACT ACT TTA GGC TAT CTA ACT TGT 780
GGT TTT CGT ATT GTC ATG TTA ACG AAA TTT 810
GAC GAA GAG ACT TTT TTA AAA ACA CTG CAA 840
GAT TAC AAA TGT TCA AGC GTT ATT CTT GTA 870
CCG ACT TTG TTT GCA ATT CTT AAT AGA AGT 900
GAA TTA CTC GAT AAA TAT GAT TTA TCA AAT 930
TTA GTT GAA ATT GCA TCT GGC GGA GCA CCT 960

990
TTA TCT AAA GAA ATT GGT GAA GCT GTT GCT
1020
AGA CGT TTT AAT TTA CCG GGT GTT CGT CAA
1050
GGC TAT GGT TTA ACA GAA ACA ACC TCT GCA
1080
ATT ATT ATC ACA CCG GAA GGC GAT GAT AAA
1110
CCA GGT GCT TCT GGC AAA GTT GTG CCA TTA
1140
TTT AAA GCA AAA GTT ATC GAT CTT GAT ACT
1170
AAA AAA ACT TTG GGC CCG AAC AGA CGT GGA
1200
GAA GTT TGT GTA AAG GGT CCT ATG CTT ATG
1230
AAA GGT TAT GTA GAT AAT CCA GAA GCA ACA
1260
AGA GAA ATC ATA GAT GAA GAA GGT TGG TTG
1290
CAC ACA GGA GAT ATT GGG TAT TAC GAT GAA
1320
GAA AAA CAT TTC TTT ATC GTG GAT CGT TTG
1350
AAG TCT TTA ATC AAA TAC AAA GGA TAT CAA
1380
GTA CCA CCT GCT GAA TTA GAA TCT GTT CTT
1410
TTG CAA CAT CCA AAT ATT TTT GAT GCC GGC
1440
GTT GCT GGC GTT CCA GAT CCT ATA GCT GGT
1470
GAG CTT CCG GGA GCT GTT GTT GTA CTT GAA
1500
AAA GGA AAA TCT ATG ACT GAA AAA GAA GTA
1530
ATG GAT TAC GTT GCT AGT CAA GTT TCA AAT
1560
GCA AAA CGT TTG CGT GGT GGT GTC CGT TTT

1590
GTG GAC GAA GTA CCT AAA GGT CTC ACT GGT
1620
AAA ATT GAC GGT AAA GCA ATT AGA GAA ATA
CTG AAG AAA CCA GTT GCT AAG ATG

4. Neue rekombinante DNA, umfassend eine Vektor-DNA, in die ein Gen gemäß den Ansprüchen 1 bis 3, das für von Luciola lateralis abgeleitete Luciferase codiert, eingeführt ist.

5. Neue rekombinante DNA gemäß Anspruch 4, dadurch gekennzeichent, daß die Vektor-DNA Plasmid pUC119 ist.

6. Verfahren zur Herstellung einer Luciferase, dadurch gekennzeichnet, daß man in einem Medium einen Mikroorganismus der Gattung Escherichia, der eine rekombinante DNA gemäß Anspruch 4 trägt, die erhalten wurde durch Einführung eines Gens, das für von Luciola lateralis abgeleitete Luciferase codiert, in eine Vektor-DNA kultiviert und die Luciferase aus der Kultur gewinnt.

7. Verfahren zur Herstellung einer Luciferase gemäß Anspruch 6, dadurch gekennzeichnet, daß die Vektor-DNA Plasmid pUC119 ist.

## Revendications

1. Gène de luciférase dérivé de Luciola lateralis, défini par la carte de restriction décrite ci-dessous:

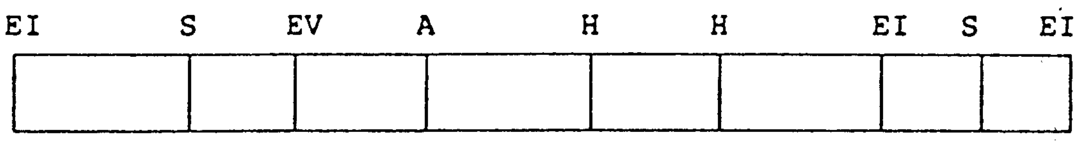


dans laquelle EI représente EcoRI, S représente Ssp I, EV représente Eco RV, A représente Apa I et H représente Hpa I.

2. Gène de luciférase selon la revendication 1, qui code la séquence d'acides aminés présentée ci-dessous:

10
Met Glu Asn Met Glu Asn Asp Glu Asn Ile
20
Val Tyr Gly Pro Glu Pro Phe Tyr Pro Ile
30
Glu Glu Gly Ser Ala Gly Ala Gln Leu Arg
40
Lys Tyr Met Asp Arg Tyr Ala Lys Leu Gly
50
Ala Ile Ala Phe Thr Asn Ala Leu Thr Gly
60
Val Asp Tyr Thr Tyr Ala Glu Tyr Leu Glu
70
Lys Ser Cys Cys Leu Gly Glu Ala Leu Lys
80
Asn Tyr Gly Leu Val Val Asp Gly Arg Ile
90
Ala Leu Cys Ser Glu Asn Cys Glu Glu Phe

100
Phe Ile Pro Val Leu Ala Gly Leu Phe Ile
110
Gly Val Gly Val Ala Pro Thr Asn Glu Ile
120
Tyr Thr Leu Arg Glu Leu Val His Ser Leu
130
Gly Ile Ser Lys Pro Thr Ile Val Phe Ser
140
Ser Lys Lys Gly Leu Asp Lys Val Ile Thr
150
Val Gln Lys Thr Val Thr Ala Ile Lys Thr
160
Ile Val Ile Leu Asp Ser Lys Val Asp Tyr
170
Arg Gly Tyr Gln Ser Met Asp Asn Phe Ile
180
Lys Lys Asn Thr Pro Gln Gly Phe Lys Gly
190
Ser Ser Phe Lys Thr Val Glu Val Asn Arg
200
Lys Glu Gln Val Ala Leu Ile Met Asn Ser
210
Ser Gly Ser Thr Gly Leu Pro Lys Gly Val
220
Gln Leu Thr His Glu Asn Ala Val Thr Arg
230
Phe Ser His Ala Arg Asp Pro Ile Tyr Gly
240
Asn Gln Val Ser Pro Gly Thr Ala Ile Leu
250
Thr Val Val Pro Phe His His Gly Phe Gly
260
Met Phe Thr Thr Leu Gly Tyr Leu Thr Cys

270

Gly Phe Arg Ile Val Met Leu Thr Lys Phe

280

Asp Glu Glu Thr Phe Leu Lys Thr Leu Gln

290

Asp Tyr Lys Cys Ser Ser Val Ile Leu Val

300

Pro Thr Leu Phe Ala Ile Leu Asn Arg Ser

310

Glu Leu Leu Asp Lys Tyr Asp Leu Ser Asn

320

Leu Val Glu Ile Ala Ser Gly Gly Ala Pro

330

Leu Ser Lys Glu Ile Gly Glu Ala Val Ala

340

Arg Arg Phe Asn Leu Pro Gly Val Arg Gln

350

Gly Tyr Gly Leu Thr Glu Thr Thr Ser Ala

360

Ile Ile Ile Thr Pro Glu Gly Asp Asp Lys

370

Pro Gly Ala Ser Gly Lys Val Val Pro Leu

380

Phe Lys Ala Lys Val Ile Asp Leu Asp Thr

390

Lys Lys Thr Leu Gly Pro Asn Arg Arg Gly

400

Glu Val Cys Val Lys Gly Pro Met Leu Met

410

Lys Gly Tyr Val Asp Asn Pro Glu Ala Thr

420

Arg Glu Ile Ile Asp Glu Glu Gly Trp Leu

430

His Thr Gly Asp Ile Gly Tyr Tyr Asp Glu

440
Glu Lys His Phe Phe Ile Val Asp Arg Leu
450
Lys Ser Leu Ile Lys Tyr Lys Gly Tyr Gln
460
Val Pro Pro Ala Glu Leu Glu Ser val Leu
470
Leu Gln His Pro Asn Ile Phe Asp Ala Gly
480
Val Ala Gly Val Pro Asp Pro Ile Ala Gly
490
Glu Leu Pro Gly Ala Val Val Val Leu Glu
500
Lys Gly Lys Ser Met Thr Glu Lys Glu Val
510
Met Asp Tyr Val Ala Ser Gln Val Ser Asn
520
Ala Lys Arg Leu Arg Gly Gly Val Arg Phe
530
Val Asp Glu Val Pro Lys Gly Leu Thr Gly
540
Lys Ile Asp Gly Lys Ala Ile Arg Glu Ile

Leu Lys Lys Pro Val Ala Lys Met

3. Gène de luciférase selon la revendication 1 ou 2 qui est représenté par la séquence nucléotidique présentée ci-dessous:

30
ATG GAA AAC ATG GAG AAC GAT GAA AAT ATT
60
GTG TAT GGT CCT GAA CCA TTT TAC CCT ATT
90
GAA GAG GGA TCT GCT GGA GCA CAA TTG CGC

120

AAG TAT ATG GAT CGA TAT GCA AAA CTT GGA

150

GCA ATT GCT TTT ACT AAC GCA CTT ACC GGT

180

GTC GAT TAT ACG TAC GCC GAA TAC TTA GAA

210

AAA TCA TGC TGT CTA GGA GAG GCT TTA AAG

240

AAT TAT GGT TTG GTT GTT GAT GGA AGA ATT

270

GCG TTA TGC AGT GAA AAC TGT GAA GAA TTC

300

TTT ATT CCT GTA TTA GCC GGT TTA TTT ATA

330

GGT GTC GGT GTG GCT CCA ACT AAT GAG ATT

360

TAC ACT CTA CGT GAA TTG GTT CAC AGT TTA

390

GGC ATC TCT AAG CCA ACA ATT GTA TTT AGT

420

TCT AAA AAA GGA TTA GAT AAA GTT ATA ACT

450

GTA CAA AAA ACG GTA ACT GCT ATT AAA ACC

480

ATT GTT ATA TTG GAC AGC AAA GTG GAT TAT

510

AGA GGT TAT CAA TCC ATG GAC AAC TTT ATT

540

AAA AAA AAC ACT CCA CAA GGT TTC AAA GGA

570

TCA AGT TTT AAA ACT GTA GAA GTT AAC CGC

600

AAA GAA CAA GTT GCT CTT ATA ATG AAC TCT

630
TCG GGT TCA ACC GGT TTG CCA AAA GGT GTG
660
CAA CTT ACT CAT GAA AAT GCA GTC ACT AGA
690
TTT TCT CAC GCT AGA GAT CCA ATT TAT GGA
720
AAC CAA GTT TCA CCA GGC ACG GCT ATT TTA
750
ACT GTA GTA CCA TTC CAT CAT GGT TTT GGT
780
ATG TTT ACT ACT TTA GGC TAT CTA ACT TGT
810
GGT TTT CGT ATT GTC ATG TTA ACG AAA TTT
840
GAC GAA GAG ACT TTT TTA AAA ACA CTG CAA
870
GAT TAC AAA TGT TCA AGC GTT ATT CTT GTA
900
CCG ACT TTG TTT GCA ATT CTT AAT AGA AGT
930
GAA TTA CTC GAT AAA TAT GAT TTA TCA AAT
960
TTA GTT GAA ATT GCA TCT GGC GGA GCA CCT
990
TTA TCT AAA GAA ATT GGT GAA GCT GTT GCT
1020
AGA CGT TTT AAT TTA CCG GGT GTT CGT CAA
1050
GGC TAT GGT TTA ACA GAA ACA ACC TCT GCA
1080
ATT ATT ATC ACA CCG GAA GGC GAT GAT AAA
1110
CCA GGT GCT TCT GGC AAA GTT GTG CAA TTA

1140
TTT AAA GCA AAA GTT ATC GAT CTT GAT ACT
1170
AAA AAA ACT TTG GGC CCG AAC AGA CGT GGA
1200
GAA GTT TGT GTA AAG GGT CCT ATG CTT ATG
1230
AAA GGT TAT GTA GAT AAT CCA GAA GCA ACA
1260
AGA GAA ATC ATA GAT GAA GAA GGT TGG TTG
1290
CAC ACA GGA GAT ATT GGG TAT TAC GAT GAA
1320
GAA AAA CAT TTC TTT ATC GTG GAT CGT TTG
1350
AAG TCT TTA ATC AAA TAC AAA GGA TAT CAA
1380
GTA CCA CCT GCT GAA TTA GAA TCT GTT CTT
1410
TTG CAA CAT CCA AAT ATT TTT GAT GCC GGC
1440
GTT GCT GGC GTT CCA GAT CCT ATA GCT GGT
1470
GAG CTT CCG GGA GCT GTT GTT GTA CTT GAA
1500
AAA GGA AAA TCT ATG ACT GAA AAA GAA GTA
1530
ATG GAT TAC GTT GCT AGT CAA GTT TCA AAT
1560
GCA AAA CGT TTG CGT GGT GGT GTC CGT TTT
1590
GTG GAC GAA GTA CCT AAA GGT CTC ACT GGT
1620
AAA ATT GAC GGT AAA GCA ATT AGA GAA ATA

CTG AAG AAA CCA GTT GCT AAG ATG

4. Nouvel ADN recombiné comprenant un ADN vecteur dans lequel est inséré un gène codant la luciférase dérivée de Luciola lateralis selon les revendications 1 à 3.

5. Nouvel ADN recombiné selon la revendication 4, dans lequel ledit ADN vecteur est le plasmide pUC119.

6. Procédé de production d'une luciférase qui comprend la culture dans un milieu d'un micro-organisme appartenant au genre Eschirichia et protant un ADN recombiné selon la revendication 4 obtenu par insertion d'un gène codant la luciférase dérivée de Luciola lateralis dans un ADN vecteur et l'obtention de ladite luciférase à partir de la culture.

7. Procédé de production d'une luciférase selon la revendication 6, dans lequel ledit ADN vecteur est le plasmide pUC119.

FIG. 1

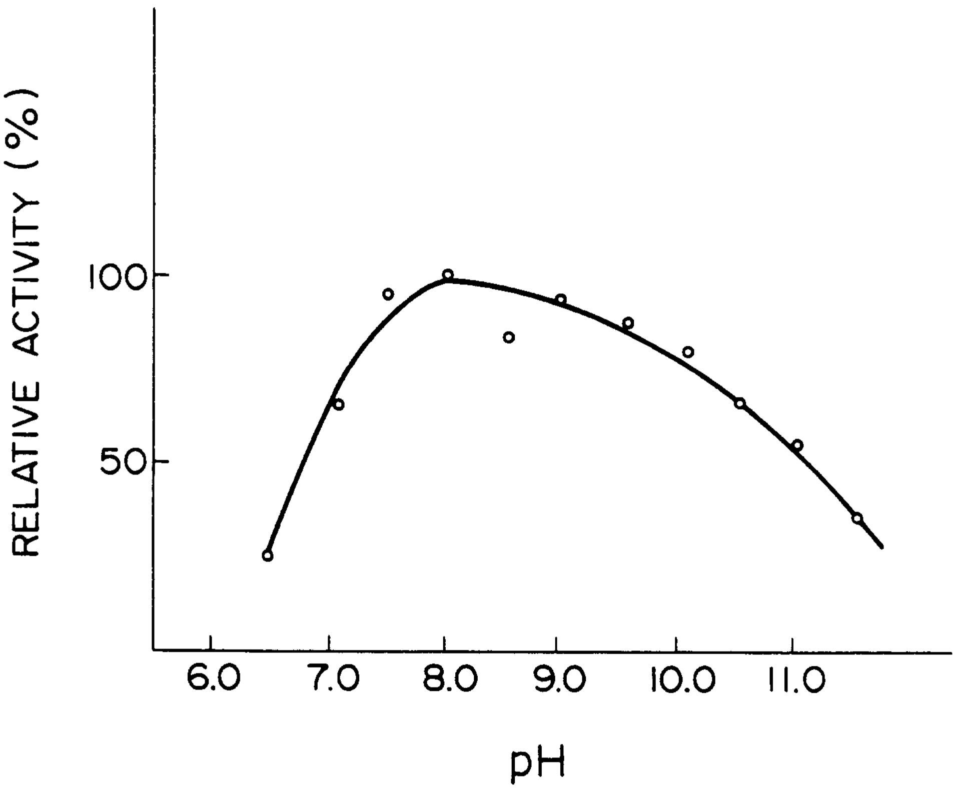

FIG. 2

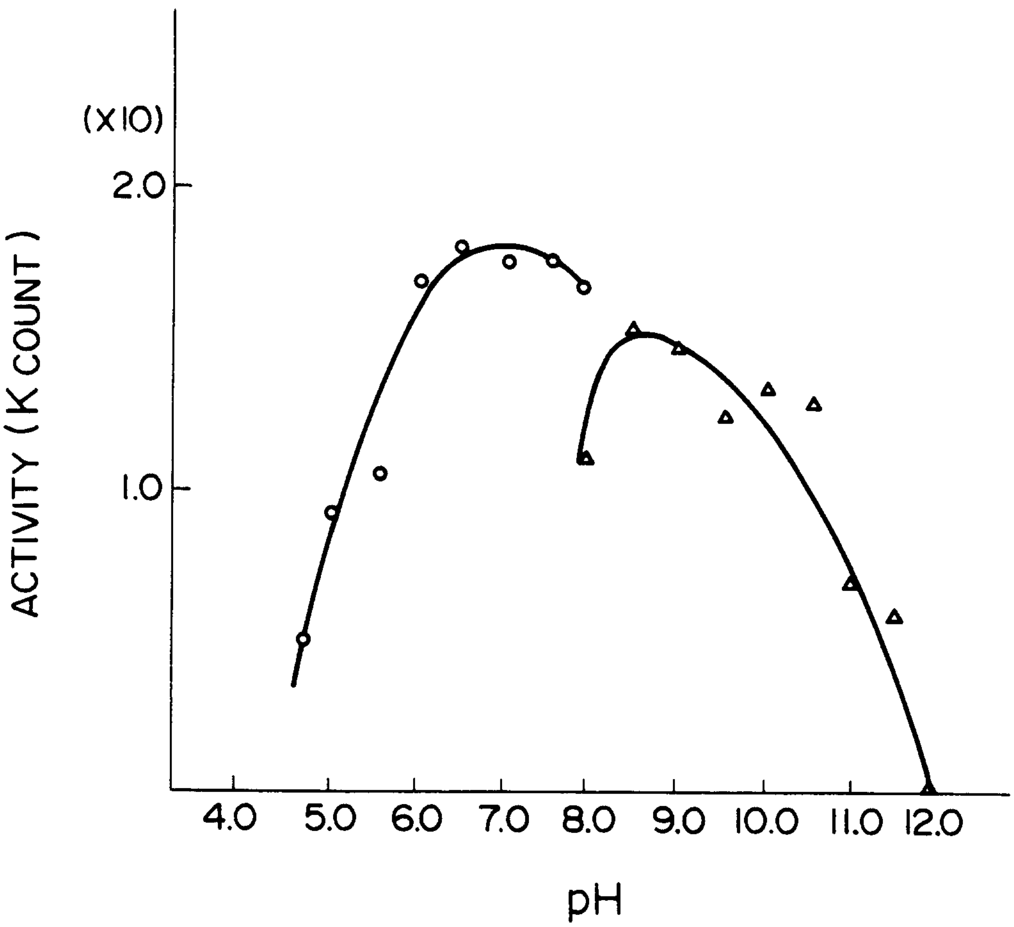

FIG. 3

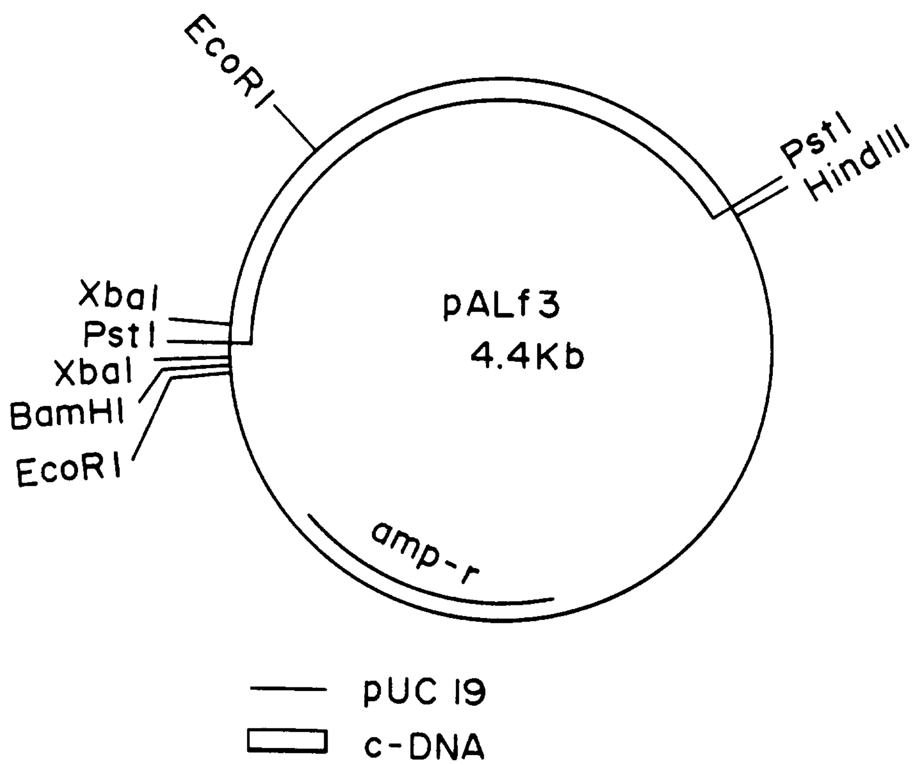
EcoRI
PstI
HindIII
XbaI
PstI
XbaI
BamHI
EcoRI
pALf3
4.4Kb
amp-r
pUC 19
c-DNA

FIG. 4

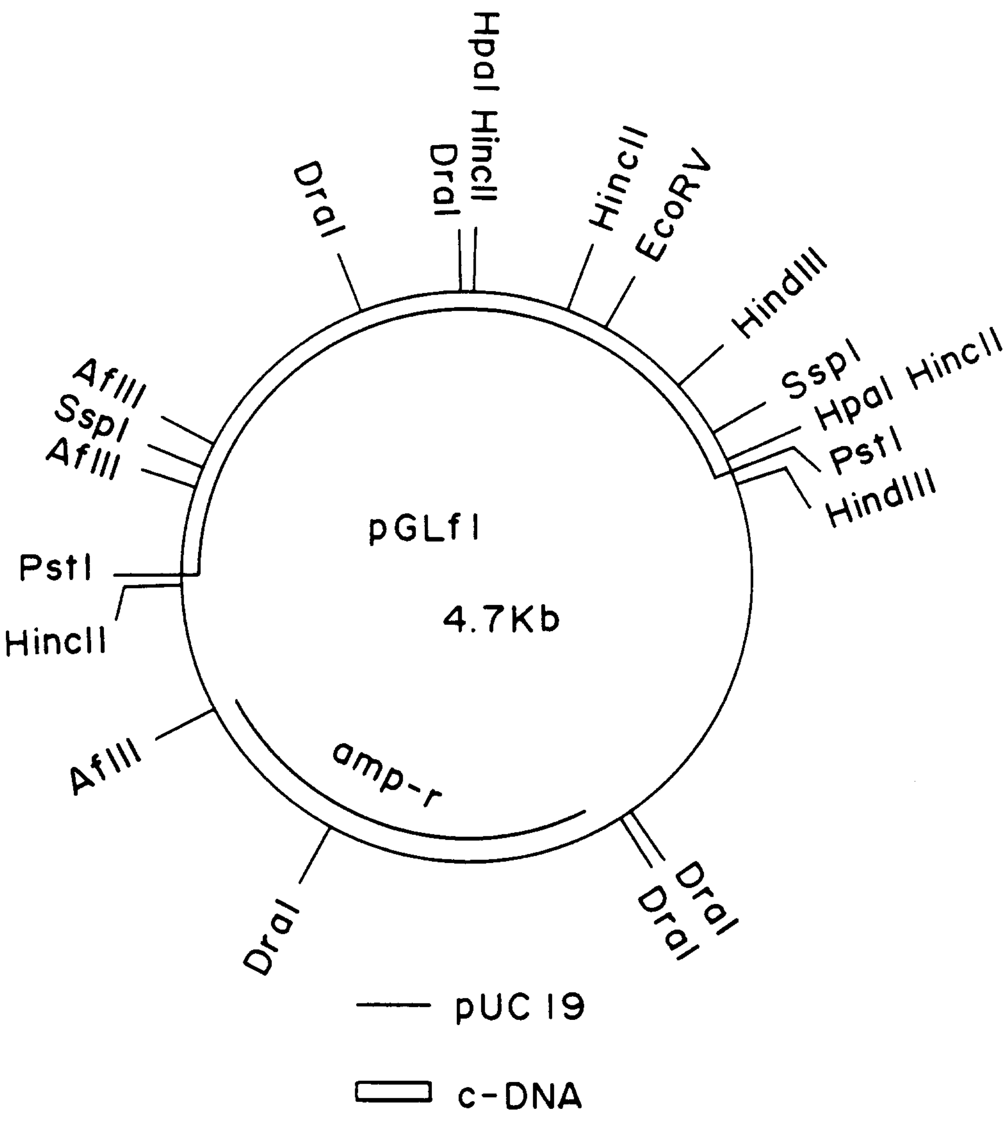
HpaI HincII
DraI
DraI
HincII
EcoRV
HindIII
SspI
HpaI HincII
PstI
HindIII
AflII
SspI
AflII
PstI
HincII
pGLf1
4.7Kb
amp-r
AflII
DraI
DraI
DraI
pUC 19
c-DNA

FIG. 5

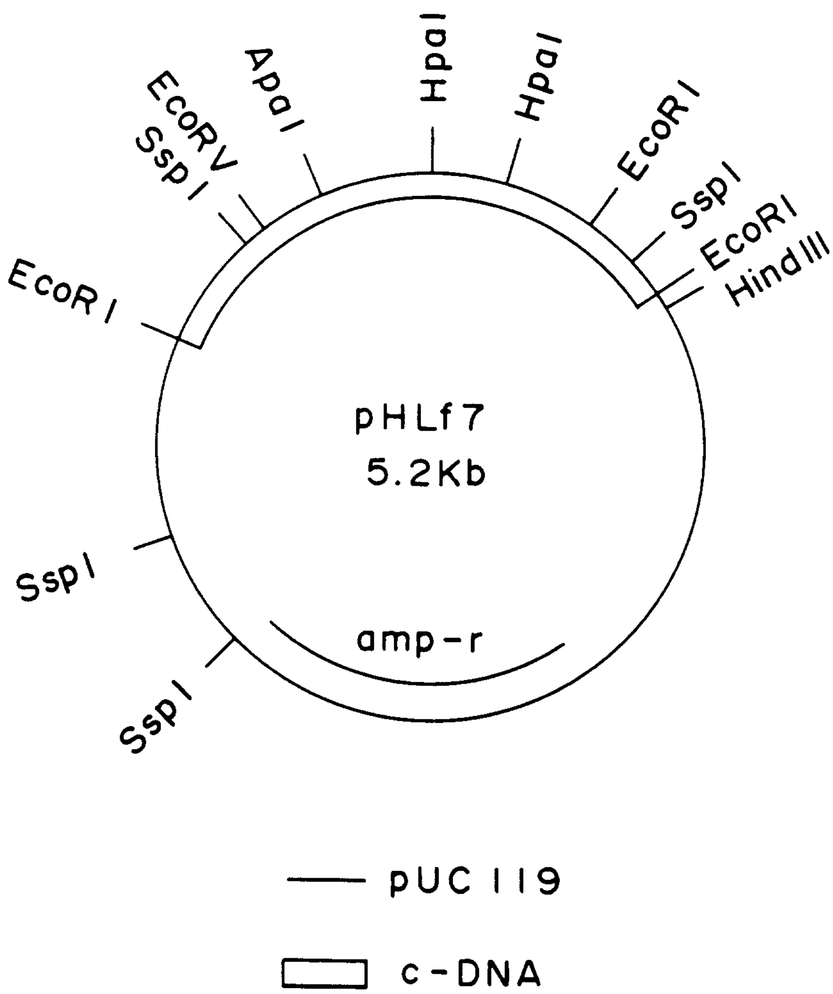
HpaI
HpaI
ApaI
EcoRV
SspI
EcoRI
EcoRI
SspI
EcoRI
HindIII
pHLf7
5.2Kb
SspI
SspI
amp-r
pUC 119
c-DNA

# FIG. 6A

| | | | | | | | | | |
|---|---|---|---|---|---|---|---|---|---|
| | | | | | | | | | 30 |
| ATG | GAA | AAC | ATG | GAG | AAC | GAT | GAA | AAT | ATT |
| | | | | | | | | | 60 |
| GTG | TAT | GGT | CCT | GAA | CCA | TTT | TAC | CCT | ATT |
| | | | | | | | | | 90 |
| GAA | GAG | GGA | TCT | GCT | GGA | GCA | CAA | TTG | CGC |
| | | | | | | | | | 120 |
| AAG | TAT | ATG | GAT | CGA | TAT | GCA | AAA | CTT | GGA |
| | | | | | | | | | 150 |
| GCA | ATT | GCT | TTT | ACT | AAC | GCA | CTT | ACC | GGT |
| | | | | | | | | | 180 |
| GTC | GAT | TAT | ACG | TAC | GCC | GAA | TAC | TTA | GAA |
| | | | | | | | | | 210 |
| AAA | TCA | TGC | TGT | CTA | GGA | GAG | GCT | TTA | AAG |
| | | | | | | | | | 240 |
| AAT | TAT | GGT | TTG | GTT | GTT | GAT | GGA | AGA | ATT |
| | | | | | | | | | 270 |
| GCG | TTA | TGC | AGT | GAA | AAC | TGT | GAA | GAA | TTC |
| | | | | | | | | | 300 |
| TTT | ATT | CCT | GTA | TTA | GCC | GGT | TTA | TTT | ATA |
| | | | | | | | | | 330 |
| GGT | GTC | GGT | GTG | GCT | CCA | ACT | AAT | GAG | ATT |
| | | | | | | | | | 360 |
| TAC | ACT | CTA | CGT | GAA | TTG | GTT | CAC | AGT | TTA |
| | | | | | | | | | 390 |
| GGC | ATC | TCT | AAG | CCA | ACA | ATT | GTA | TTT | AGT |
| | | | | | | | | | 420 |
| TCT | AAA | AAA | GGA | TTA | GAT | AAA | GTT | ATA | ACT |
| | | | | | | | | | 450 |
| GTA | CAA | AAA | ACG | GTA | ACT | GCT | ATT | AAA | ACC |
| | | | | | | | | | 480 |
| ATT | GTT | ATA | TTG | GAC | AGC | AAA | GTG | GAT | TAT |
| | | | | | | | | | 510 |
| AGA | GGT | TAT | CAA | TCC | ATG | GAC | AAC | TTT | ATT |
| | | | | | | | | | 540 |
| AAA | AAA | AAC | ACT | CCA | CAA | GGT | TTC | AAA | GGA |
| | | | | | | | | | 570 |
| TCA | AGT | TTT | AAA | ACT | GTA | GAA | GTT | AAC | CGC |

# FIG. 6B

| | | | | | | | | | |
|---|---|---|---|---|---|---|---|---|---|
| | | | | | | | | | 600 |
| AAA | GAA | CAA | GTT | GCT | CTT | ATA | ATG | AAC | TCT |
| | | | | | | | | | 630 |
| TCG | GGT | TCA | ACC | GGT | TTG | CCA | AAA | GGT | GTG |
| | | | | | | | | | 660 |
| CAA | CTT | ACT | CAT | GAA | AAT | GCA | GTC | ACT | AGA |
| | | | | | | | | | 690 |
| TTT | TCT | CAC | GCT | AGA | GAT | CCA | ATT | TAT | GGA |
| | | | | | | | | | 720 |
| AAC | CAA | GTT | TCA | CCA | GGC | ACG | GCT | ATT | TTA |
| | | | | | | | | | 750 |
| ACT | GTA | GTA | CCA | TTC | CAT | CAT | GGT | TTT | GGT |
| | | | | | | | | | 780 |
| ATG | TTT | ACT | ACT | TTA | GGC | TAT | CTA | ACT | TGT |
| | | | | | | | | | 810 |
| GGT | TTT | CGT | ATT | GTC | ATG | TTA | ACG | AAA | TTT |
| | | | | | | | | | 840 |
| GAC | GAA | GAG | ACT | TTT | TTA | AAA | ACA | CTG | CAA |
| | | | | | | | | | 870 |
| GAT | TAC | AAA | TGT | TCA | AGC | GTT | ATT | CTT | GTA |
| | | | | | | | | | 900 |
| CCG | ACT | TTG | TTT | GCA | ATT | CTT | AAT | AGA | AGT |
| | | | | | | | | | 930 |
| GAA | TTA | CTC | GAT | AAA | TAT | GAT | TTA | TCA | AAT |
| | | | | | | | | | 960 |
| TTA | GTT | GAA | ATT | GCA | TCT | GGC | GGA | GCA | CCT |
| | | | | | | | | | 990 |
| TTA | TCT | AAA | GAA | ATT | GGT | GAA | GCT | GTT | GCT |
| | | | | | | | | | 1020 |
| AGA | CGT | TTT | AAT | TTA | CCG | GGT | GTT | CGT | CAA |
| | | | | | | | | | 1050 |
| GGC | TAT | GGT | TTA | ACA | GAA | ACA | ACC | TCT | GCA |
| | | | | | | | | | 1080 |
| ATT | ATT | ATC | ACA | CCG | GAA | GGC | GAT | GAT | AAA |
| | | | | | | | | | 1110 |
| CCA | GGT | GCT | TCT | GGC | AAA | GTT | GTG | CCA | TTA |
| | | | | | | | | | 1140 |
| TTT | AAA | GCA | AAA | GTT | ATC | GAT | CTT | GAT | ACT |

# FIG. 6C

AAA AAA ACT TTG GGC CCG AAC AGA CGT GGA 1170

GAA GTT TGT GTA AAG GGT CCT ATG CTT ATG 1200

AAA GGT TAT GTA GAT AAT CCA GAA GCA ACA 1230

AGA GAA ATC ATA GAT GAA GAA GGT TGG TTG 1260

CAC ACA GGA GAT ATT GGG TAT TAC GAT GAA 1290

GAA AAA CAT TTC TTT ATC GTG GAT CGT TTG 1320

AAG TCT TTA ATC AAA TAC AAA GGA TAT CAA 1350

GTA CCA CCT GCT GAA TTA GAA TCT GTT CTT 1380

TTG CAA CAT CCA AAT ATT TTT GAT GCC GGC 1410

GTT GCT GGC GTT CCA GAT CCT ATA GCT GGT 1440

GAG CTT CCG GGA GCT GTT GTT GTA CTT GAA 1470

AAA GGA AAA TCT ATG ACT GAA AAA GAA GTA 1500

ATG GAT TAC GTT GCT AGT CAA GTT TCA AAT 1530

GCA AAA CGT TTG CGT GGT GGT GTC CGT TTT 1560

GTG GAC GAA GTA CCT AAA GGT CTC ACT GGT 1590

AAA ATT GAC GGT AAA GCA ATT AGA GAA ATA 1620

CTG AAG AAA CCA GTT GCT AAG ATG

# FIG. 7A

Met Glu Asn Met Glu Asn Asp Glu Asn Ile 10
Val Tyr Gly Pro Glu Pro Phe Tyr Pro Ile 20
Glu Glu Gly Ser Ala Gly Ala Gln Leu Arg 30
Lys Tyr Met Asp Arg Tyr Ala Lys Leu Gly 40
Ala Ile Ala Phe Thr Asn Ala Leu Thr Gly 50
Val Asp Tyr Thr Tyr Ala Glu Tyr Leu Glu 60
Lys Ser Cys Cys Leu Gly Glu Ala Leu Lys 70
Asn Tyr Gly Leu Val Val Asp Gly Arg Ile 80
Ala Leu Cys Ser Glu Asn Cys Glu Glu Phe 90
Phe Ile Pro Val Leu Ala Gly Leu Phe Ile 100
Gly Val Gly Val Ala Pro Thr Asn Glu Ile 110
Tyr Thr Leu Arg Glu Leu Val His Ser Leu 120
Gly Ile Ser Lys Pro Thr Ile Val Phe Ser 130
Ser Lys Lys Gly Leu Asp Lys Val Ile Thr 140
Val Gln Lys Thr Val Thr Ala Ile Lys Thr 150
Ile Val Ile Leu Asp Ser Lys Val Asp Tyr 160
Arg Gly Tyr Gln Ser Met Asp Asn Phe Ile 170
Lys Lys Asn Thr Pro Gln Gly Phe Lys Gly 180
Ser Ser Phe Lys Thr Val Glu Val Asn Arg 190

# FIG. 7B

| | | | | | | | | | |
|---|---|---|---|---|---|---|---|---|---|
| | | | | | | | | | 200 |
| Lys | Glu | Gln | Val | Ala | Leu | Ile | Met | Asn | Ser |
| | | | | | | | | | 210 |
| Ser | Gly | Ser | Thr | Gly | Leu | Pro | Lys | Gly | Val |
| | | | | | | | | | 220 |
| Gln | Leu | Thr | His | Glu | Asn | Ala | Val | Thr | Arg |
| | | | | | | | | | 230 |
| Phe | Ser | His | Ala | Arg | Asp | Pro | Ile | Tyr | Gly |
| | | | | | | | | | 240 |
| Asn | Gln | Val | Ser | Pro | Gly | Thr | Ala | Ile | Leu |
| | | | | | | | | | 250 |
| Thr | Val | Val | Pro | Phe | His | His | Gly | Phe | Gly |
| | | | | | | | | | 260 |
| Met | Phe | Thr | Thr | Leu | Gly | Tyr | Leu | Thr | Cys |
| | | | | | | | | | 270 |
| Gly | Phe | Arg | Ile | Val | Met | Leu | Thr | Lys | Phe |
| | | | | | | | | | 280 |
| Asp | Glu | Glu | Thr | Phe | Leu | Lys | Thr | Leu | Gln |
| | | | | | | | | | 290 |
| Asp | Tyr | Lys | Cys | Ser | Ser | Val | Ile | Leu | Val |
| | | | | | | | | | 300 |
| Pro | Thr | Leu | Phe | Ala | Ile | Leu | Asn | Arg | Ser |
| | | | | | | | | | 310 |
| Glu | Leu | Leu | Asp | Lys | Tyr | Asp | Leu | Ser | Asn |
| | | | | | | | | | 320 |
| Leu | Val | Glu | Ile | Ala | Ser | Gly | Gly | Ala | Pro |
| | | | | | | | | | 330 |
| Leu | Ser | Lys | Glu | Ile | Gly | Glu | Ala | Val | Ala |
| | | | | | | | | | 340 |
| Arg | Arg | Phe | Asn | Leu | Pro | Gly | Val | Arg | Gln |
| | | | | | | | | | 350 |
| Gly | Tyr | Gly | Leu | Thr | Glu | Thr | Thr | Ser | Ala |
| | | | | | | | | | 360 |
| Ile | Ile | Ile | Thr | Pro | Glu | Gly | Asp | Asp | Lys |
| | | | | | | | | | 370 |
| Pro | Gly | Ala | Ser | Gly | Lys | Val | Val | Pro | Leu |
| | | | | | | | | | 380 |
| Phe | Lys | Ala | Lys | Val | Ile | Asp | Leu | Asp | Thr |

# FIG. 7C

390
Lys Lys Thr Leu Gly Pro Asn Arg Arg Gly
400
Glu Val Cys Val Lys Gly Pro Met Leu Met
410
Lys Gly Tyr Val Asp Asn Pro Glu Ala Thr
420
Arg Glu Ile Ile Asp Glu Glu Gly Trp Leu
430
His Thr Gly Asp Ile Gly Tyr Tyr Asp Glu
440
Glu Lys His Phe Phe Ile Val Asp Arg Leu
450
Lys Ser Leu Ile Lys Tyr Lys Gly Tyr Gln
460
Val Pro Pro Ala Glu Leu Glu Ser Val Leu
470
Leu Gln His Pro Asn Ile Phe Asp Ala Gly
480
Val Ala Gly Val Pro Asp Pro Ile Ala Gly
490
Glu Leu Pro Gly Ala Val Val Val Leu Glu
500
Lys Gly Lys Ser Met Thr Glu Lys Glu Val
510
Met Asp Tyr Val Ala Ser Gln Val Ser Asn
520
Ala Lys Arg Leu Arg Gly Gly Val Arg Phe
530
Val Asp Glu Val Pro Lys Gly Leu Thr Gly
540
Lys Ile Asp Gly Lys Ala Ile Arg Glu Ile

Leu Lys Lys Pro Val Ala Lys Met

# FIG. 8A

```
                                     10
ATG GAA AAC ATG GAG AAC GAT GAA AAT ATT
Met Glu Asn Met Glu Asn Asp Glu Asn Ile
                                     20
GTG TAT GGT CCT GAA CCA TTT TAC CCT ATT
Val Tyr Gly Pro Glu Pro Phe Tyr Pro Ile
                                     30
GAA GAG GGA TCT GCT GGA GCA CAA TTG CGC
Glu Glu Gly Ser Ala Gly Ala Gln Leu Arg
                                     40
AAG TAT ATG GAT CGA TAT GCA AAA CTT GGA
Lys Tyr Met Asp Arg Tyr Ala Lys Leu Gly
                                     50
GCA ATT GCT TTT ACT AAC GCA CTT ACC GGT
Ala Ile Ala Phe Thr Asn Ala Leu Thr Gly
                                     60
GTC GAT TAT ACG TAC GCC GAA TAC TTA GAA
Val Asp Tyr Thr Tyr Ala Glu Tyr Leu Glu
                                     70
AAA TCA TGC TGT CTA GGA GAG GCT TTA AAG
Lys Ser Cys Cys Leu Gly Glu Ala Leu Lys
                                     80
AAT TAT GGT TTG GTT GTT GAT GGA AGA ATT
Asn Tyr Gly Leu Val Val Asp Gly Arg Ile
                                     90
GCG TTA TGC AGT GAA AAC TGT GAA GAA TTC
Ala Leu Cys Ser Glu Asn Cys Glu Glu Phe
                                    100
TTT ATT CCT GTA TTA GCC GGT TTA TTT ATA
Phe Ile Pro Val Leu Ala Gly Leu Phe Ile
                                    110
GGT GTC GGT GTG GCT CCA ACT AAT GAG ATT
Gly Val Gly Val Ala Pro Thr Asn Glu Ile
                                    120
TAC ACT CTA CGT GAA TTG GTT CAC AGT TTA
Tyr Thr Leu Arg Glu Leu Val His Ser Leu
```

# FIG. 8B

```
                                            130
GGC ATC TCT AAG CCA ACA ATT GTA TTT AGT
Gly Ile Ser Lys Pro Thr Ile Val Phe Ser
                                            140
TCT AAA AAA GGA TTA GAT AAA GTT ATA ACT
Ser Lys Lys Gly Leu Asp Lys Val Ile Thr
                                            150
GTA CAA AAA ACG GTA ACT GCT ATT AAA ACC
Val Gln Lys Thr Val Thr Ala Ile Lys Thr
                                            160
ATT GTT ATA TTG GAC AGC AAA GTG GAT TAT
Ile Val Ile Leu Asp Ser Lys Val Asp Tyr
                                            170
AGA GGT TAT CAA TCC ATG GAC AAC TTT ATT
Arg Gly Tyr Gln Ser Met Asp Asn Phe Ile
                                            180
AAA AAA AAC ACT CCA CAA GGT TTC AAA GGA
Lys Lys Asn Thr Pro Gln Gly Phe Lys Gly
                                            190
TCA AGT TTT AAA ACT GTA GAA GTT AAC CGC
Ser Ser Phe Lys Thr Val Glu Val Asn Arg
                                            200
AAA GAA CAA GTT GCT CTT ATA ATG AAC TCT
Lys Glu Gln Val Ala Leu Ile Met Asn Ser
                                            210
TCG GGT TCA ACC GGT TTG CCA AAA GGT GTG
Ser Gly Ser Thr Gly Leu Pro Lys Gly Val
                                            220
CAA CTT ACT CAT GAA AAT GCA GTC ACT AGA
Gln Leu Thr His Glu Asn Ala Val Thr Arg
                                            230
TTT TCT CAC GCT AGA GAT CCA ATT TAT GGA
Phe Ser His Ala Arg Asp Pro Ile Tyr Gly
                                            240
AAC CAA GTT TCA CCA GGC ACG GCT ATT TTA
Asn Gln Val Ser Pro Gly Thr Ala Ile Leu
```

# FIG. 8C

```
                                                 250
ACT GTA GTA CCA TTC CAT CAT GGT TTT GGT
Thr Val Val Pro Phe His His Gly Phe Gly
                                                 260
ATG TTT ACT ACT TTA GGC TAT CTA ACT TGT
Met Phe Thr Thr Leu Gly Tyr Leu Thr Cys
                                                 270
GGT TTT CGT ATT GTC ATG TTA ACG AAA TTT
Gly Phe Arg Ile Val Met Leu Thr Lys Phe
                                                 280
GAC GAA GAG ACT TTT TTA AAA ACA CTG CAA
Asp Glu Glu Thr Phe Leu Lys Thr Leu Gln
                                                 290
GAT TAC AAA TGT TCA AGC GTT ATT CTT GTA
Asp Tyr Lys Cys Ser Ser Val Ile Leu Val
                                                 300
CCG ACT TTG TTT GCA ATT CTT AAT AGA AGT
Pro Thr Leu Phe Ala Ile Leu Asn Arg Ser
                                                 310
GAA TTA CTC GAT AAA TAT GAT TTA TCA AAT
Glu Leu Leu Asp Lys Tyr Asp Leu Ser Asn
                                                 320
TTA GTT GAA ATT GCA TCT GGC GGA GCA CCT
Leu Val Glu Ile Ala Ser Gly Gly Ala Pro
                                                 330
TTA TCT AAA GAA ATT GGT GAA GCT GTT GCT
Leu Ser Lys Glu Ile Gly Glu Ala Val Ala
                                                 340
AGA CGT TTT AAT TTA CCG GGT GTT CGT CAA
Arg Arg Phe Asn Leu Pro Gly Val Arg Gln
                                                 350
GGC TAT GGT TTA ACA GAA ACA ACC TCT GCA
Gly Tyr Gly Leu Thr Glu Thr Thr Ser Ala
                                                 360
ATT ATT ATC ACA CCG GAA GGC GAT GAT AAA
Ile Ile Ile Thr Pro Glu Gly Asp Asp Lys
```

# FIG. 8D

370
CCA GGT GCT TCT GGC AAA GTT GTG CCA TTA
Pro Gly Ala Ser Gly Lys Val Val Pro Leu

380
TTT AAA GCA AAA GTT ATC GAT CTT GAT ACT
Phe Lys Ala Lys Val Ile Asp Leu Asp Thr

390
AAA AAA ACT TTG GGC CCG AAC AGA CGT GGA
Lys Lys Thr Leu Gly Pro Asn Arg Arg Gly

400
GAA GTT TGT GTA AAG GGT CCT ATG CTT ATG
Glu Val Cys Val Lys Gly Pro Met Leu Met

410
AAA GGT TAT GTA GAT AAT CCA GAA GCA ACA
Lys Gly Tyr Val Asp Asn Pro Glu Ala Thr

420
AGA GAA ATC ATA GAT GAA GAA GGT TGG TTG
Arg Glu Ile Ile Asp Glu Glu Gly Trp Leu

430
CAC ACA GGA GAT ATT GGG TAT TAC GAT GAA
His Thr Gly Asp Ile Gly Tyr Tyr Asp Glu

440
GAA AAA CAT TTC TTT ATC GTG GAT CGT TTG
Glu Lys His Phe Phe Ile Val Asp Arg Leu

450
AAG TCT TTA ATC AAA TAC AAA GGA TAT CAA
Lys Ser Leu Ile Lys Tyr Lys Gly Tyr Gln

460
GTA CCA CCT GCT GAA TTA GAA TCT GTT CTT
Val Pro Pro Ala Glu Leu Glu Ser Val Leu

470
TTG CAA CAT CCA AAT ATT TTT GAT GCC GGC
Leu Gln His Pro Asn Ile Phe Asp Ala Gly

480
GTT GCT GGC GTT CCA GAT CCT ATA GCT GGT
Val Ala Gly Val Pro Asp Pro Ile Ala Gly

# FIG. 8E

490
GAG CTT CCG GGA GCT GTT GTT GTA CTT GAA
Glu Leu Pro Gly Ala Val Val Val Leu Glu

500
AAA GGA AAA TCT ATG ACT GAA AAA GAA GTA
Lys Gly Lys Ser Met Thr Glu Lys Glu Val

510
ATG GAT TAC GTT GCT AGT CAA GTT TCA AAT
Met Asp Tyr Val Ala Ser Gln Val Ser Asn

520
GCA AAA CGT TTG CGT GGT GGT GTC CGT TTT
Ala Lys Arg Leu Arg Gly Gly Val Arg Phe

530
GTG GAC GAA GTA CCT AAA GGT CTC ACT GGT
Val Asp Glu Val Pro Lys Gly Leu Thr Gly

540
AAA ATT GAC GGT AAA GCA ATT AGA GAA ATA
Lys Ile Asp Gly Lys Ala Ile Arg Glu Ile

CTG AAG AAA CCA GTT GCT AAG ATG
Leu Lys Lys Pro Val Ala Lys Met